# EUROPEAN PATENT SPECIFICATION

(11) **EP 1 878 429 B1**
(45) Date of publication and mention of the grant of the patent: **03.08.2011**
(21) Application number: 06745734.1
(22) Date of filing: 27.04.2006
(51) Int. Cl.: A61K 31/5575, A61K 9/06, A61K 9/70, A61K 47/06, A61K 47/10, A61K 47/12, A61K 47/14, A61K 47/18, A61K 47/32, A61K 47/34, A61K 47/44, A61P 19/08, A61P 43/00

(54) **TRENADERMAL ABSORPTION PREPARATION**
PRÄPARAT ZUR TRANSDERMALEN ABSORPTION
PRÉPARATION POUR ABSORPTION TRANSDERMALE

(30) Priority: 28.04.2005 JP 2005130688
(43) Date of publication of application: 16.01.2008
(73) Proprietor: ONO PHARMACEUTICAL CO., LTD., Osaka-shi 541-8526 (JP)
(72) Inventor: NISHIURA, Akio, ONO PHARMACEUTICAL CO., LTD., Mishima-gun, Osaka 618-8585 (JP); SUGIHARA, Hikaru, ONO PHARMACEUTICAL CO., LTD., Mishima-gun, Osaka 618-8585 (JP); ARAI, Harumi, ONO PHARMACEUTICAL CO., LTD., Mishima-gun, Osaka 618-8585 (JP); KANAJI, Toshiya, ONO PHARMACEUTICAL CO., LTD., Mishima-gun, Osaka 618-8585 (JP)
(74) Representative: McCluskie, Gail Wilson
(86) International application number: PCT/JP2006/308806
(87) International publication number: WO 2006/118173

(56) References cited:
- EP-A- 0 742 011
- WO-A1-01/37877
- WO-A1-95/31190
- WO-A1-02/098396
- WO-A1-03/041717
- JP-A- 63 203 615
- JP-A- 2002 332 228
- US-A- 5 380 760
- US-A- 5 468 501
- SHIN: 'Drug Delivery System' KABUSHIKI KAISHA CMC 2001, pages 34 - 37, XP003004792
- 'Drug Delivery System' NANKODO CO., LTD. 1986, pages 98 - 103, XP003004793

## Description

The present invention relates to a percutaneous absorption preparation which comprises a methyl ester of 4-{[2-((1R,2R,3R)-3-hydroxy-2-{(1E,3S)-3-hydroxy-4-[3-(methoxymethyl)phenyl]but-1-enyl}-5-oxocyclopentyl)ethyl]sulfanyl}butanoic acid.

### Background of the Invention

Prostaglandin (to be referred to as PG hereinafter)s are compounds which show strong activities with a very small amount by broadly distributing in various organs and body fluid in the living body and have a great variety of physiological activities such as relaxation and contraction of smooth muscle, contraction and dilation of blood vessel, platelet agglutination inhibitory activity and the like. Among PGs, since PGE₂ has physiological activities such as cell protective activity, uterine contraction, pain producing activity, acceleration of digestive tract peristalsis, awakening activity, a suppressive effect on gastric acid secretion, hypotensive activity and diuretic activity, and it has been used in the prevention and treatment of various diseases.

However, since PGs including this PGE₂ are chemically very unstable, their effective administration methods are limited to intravenous administration and the like and have a possibility of side effects such as expressing hypotension, gastroenteric disorder, skin blood vessel edema (The Journal of Clinical Investigation, vol. 108, pp. 25 - 30, 2001). Therefore, studies have been carried out on the possibility of percutaneous absorption preparations, particularly patches, of PGs as new parenteral route of administration and form of administration (e.g., see Patent References 1, 2 and 3). As a characteristic of such patches, there is a merit of alleviating burdens on patients such as that home therapy is possible, pains are not accompanied by the administration; and their removal after administration is possible.

However, due to the barrier function of the skin, percutaneous absorption of a drug is generally poor, and it is difficult in many cases to deliver an amount of the drug which is necessary for expressing its pharmacological effect from the skin within a practical and limited applying area. Additionally, patches have many problems such as stability of the drug, effect, safety (expression of side effects), adhesiveness, a sense of disconfort, skin irritation (e.g. erythema, edema, itchy feeling, exanthema, pigmentation and the like) and the like.

On the other hand. 4-{[2-((1R,2R,3R)-3-hydroxy-2-{(1E,3S)-3-hydroxy-4-[3-(methoxymethyl)phenyl]but-1-enyl}-5-oxocyclopentyl)ethyl]sulfanyl}butanoic acid is a compound known as a 5-thia-ω-substituted phenyl-prostaglandin E derivative having the agonist activity for EP4 which is one of the PAGE₂ receptor subtypes. It has been disclosed that an ester form of 4-{[2-((1R,2R,3R)-3-hydroxy-2-{(1E,3S)-3-hydroxy-4-[3-(methoxymethyl)phenyl]but-1-enyl}-5-oxocyclopentyl)ethyl]sulfanyl} butanoic acid, as its prodrug form, can be used as a preventive and/or therapeutic agent for various diseases such as a bone loss disease and the like, by making it into patches for percutaneous administration or sustained release preparations for topical administration (e.g., microcapsule preparations, microsphere preparations, nanosphere preparations and the like), or as embedding type sustained-release film preparations for topical administration use by dissolving it in a bioabsorbable polymer and an organic solvent and freeze-drying the solution (e.g., see Patent References 4, 5, 6 and 7). Additionally, percutaneous administration of one of the PGE2 receptor subtypes, EP1 agonist, disclosed for the purpose of increasing bone volume (e.g., see Patent Reference 8), and was disclosed also that topical administration of an EP4-selective agonist is useful for the treatment of bone diseases (e.g., see Patent Reference 9).

However, nothing is described in these references on the specific application of an ester form of 4-{[2-((1R,2R,3R)-3-hydroxy-2-{(1E,3S)-3-hydroxy-4-[3-(methoxymethyl)phenyl]but-1-enyl}-5-oxocyclopentyl)ethyl]sulfanyl}butanoic acid as blood concentration regulating type percutaneous absorption preparations, particularly patches, and experimental confirmation thereof.
[Patent Reference 1] JP-A-58-134019
[Patent Reference 2] JP-B-07-25666
[Patent Reference 3] Japanese Patent No. 2910857
[Patent Reference 4] International Publication No. 00/003980
[Patent Reference 5] International Publication No. 01/037877
[Patent Reference 6] International Publication No. 03/009872
[Patent Reference 7] International Publication No. 03/041717
[Patent Reference 8] International Publication No. 00/051585
[Patent Reference 9] JP-A-2001-181210

### Disclosure of the Invention

### Problems to be Solved by the Invention

When systemic administration such as oral administration and intravenous administration (e.g., rapid-sequence intravenous injection, drip infusion or the like) of an ester form of 4-{[2-((1R,2R,3R)-3-hydroxy-2-{(1E,3S)-3-hydroxy-4-[3-(methoxymethyl)phenyl]but-1-enyl}-5-oxocyclopentyl)ethyl]sulfanyl}butanoic acid is carried out, it is converted into its active form, 4-{[2-((1R,2R,3R)-3-hydroxy-2-{(1E,3S)-3-hydroxy-4-[3-(methoxymethyl)phenyl]but-1-enyl}-5-oxocyclopentyl) ethyl]sulfanyl}butanoic acid, and when its blood concentration is abnormally increased, there is a possible danger of side effects such as exerting influences upon circulatory organs, such as hypotension and increase in cardiac rate and of causing diarrhea. Also, since bone repair requires time, when a bone disease is treated, it is necessary to administer the pharmaceutical preparation to the topical region a large number of times in the case of intravenous injection, which is a burden on the patient Additionally, in the case of the percutaneous administration of PGs and EP4 agonist, there is a possibility of causing skin blood vessel edema as a side effect.

The object of the present invention is to provide a blood concentration regulation type percutaneous absorption preparation comprising methyl 4-{[2-((1R, 2R, 3R)-3-hydroxy-2-{(1E,3S)-3-hydroxy-4-[3-(methoxymethyl)phenyl]but-1-enyl}-5-oxocyclopentyl)ethyl]sulfanyl}butanoate, which exerts sufficient pharmacological activity and is safe since side effects can be avoided.

### Means for Solving the Problems

With the aim of achieving the aforementioned object, the inventors of the present invention have conducted intensive studies and found as a result that a blood concentration regulation type percutaneous absorption preparation which comprises methyl 4-{[2-((1R, 2R, 3R)-3-hydroxy-2-{(1E,3S)-3-hydroxy-4-[3-(methoxymethyl)phenyl]but-1-enyl}-5-oxocyclopentyl)ethyl]sulfanyl}butanoate and a base for external prepartions, which may contain a percutaneous permeation accelerator, can continuously control blood concentration of its active form, 4-{[2-((1R,2R,3R)-3-hydroxy-2-{(1E,3S)-3-hydroxy-4-[3-(methoxymethyl)phenyl]but-1-enyl}-5-oxocyclopentyl)ethyl]sulfanyl}butanoic acid, and can suppress side effects. The base for external preparations is an adhesive that may contain an adhesion providing agent, a softening agent and/or an antioxidant.

Moreover, it was found that the blood concentration regulation type percutaneous absorption preparation of the present invention has bone repair activity. Additionally, it was found also that patches are preferable among percutaneous absorption preparations, and that side effects can be easily avoided by the sue of patches by releasing them as occasion demands.

Namely, the present invention relates to the following.
[1] A blood concentration regulation type patch, which comprises methyl 4-{[2-((1R,2R,3R)-3-hydroxy-2-{(1E,3S)-3-hydroxy-4-[3-(methoxymethyl)phenyl]but-1-enyl}-5-oxocyclopentyl)ethyl]sulfanyl}butanoate, and a percutaneous permeation accelerator is one or more substances selected from the group consisting of isopropyl myristate, crotamiton, oleic and oleyl alcohol and an adhesive which may contain an adhesion providing agent, a softening agent, and/or an antioxidant.
[2] The patch according to [1], wherein the adhesive is one or more substances selected from the group consisting of a styrene-isoprene-styrene block copolymer, an acrylic acid ester resin and an acrylic system copolymer resin;
   the adhesion providing agent is one or more substances selected from a group consisting of a rosin resin, an alicyclic saturated hydrocarbon resin and a polyisobutylene;
   the softening agent is liquid paraffins; and
   the antioxidant is di-t-butylhydroxytoluene.
[3] The patch according to [2], wherein the adhesive is a styrene-isoprene-styrene block copolymer;
   the adhesion providing agent is one or more substances selected from the group consisting of a rosin resin, an alicyclic saturated hydrocarbon resin and a polyisobutylene; and
   the percutaneous permeation accelerator is oleyl alcohol.
[4] The patch according to [1], which is an agent for preventing, treating and/or advance-suppressing a bone disease.
[5] The patch according to [4], wherein the bone disease is bone fracture or vertebral body fracture.
[6] The patch according to any one of [1] to [5], which comprises (i) from about 10 parts by mass to about 200 parts by mass of a styrene-isoprene-styrene block copolymer, (ii) from about 20 parts by mass to about 300 parts by mass of an alicyclic saturated hydrocarbon resin, (iii) from about 30 parts by mass to about 500 parts by mass of liquid paraffins, (iv) from about 1 part by mass to about 100 parts by mass of a high molecular weight polyisobutylene, (v) from about 5 parts by mass to about 200 parts by mass of a low molecular weight polyisobutylene, (vi) from about 1 part by mass to about 100 parts by mass of a rosin resin and (vii) from about 0.1 part by mass to about 3 parts by mass of di-t-butylhydroxytoluene, and further comprises from about 1 part by mass to about 50 parts by mass of oleyl alcohol, based on 1 part by mass of methyl 4-{[2-((1R,2R,3R)-3-hydroxy-2-{(1E,3S)-3-hydroxy-4-[3-(methoxymethyl)phenyl]but-1-enyl}-5-oxocyclopentyl)ethyl]sulfanyl}butanoate.
[7] The patch according to [6], wherein the methyl 4-{[2-((1R,2R,3R)-3-hydroxy-2-{(1E,3S)-3-hydroxy-4-[3-(methoxymethyl)phenyl]but-1-enyl}-5-oxocyclopentyl)ethyl]sulfanyl}butanoate content per one dose is from about 0.01 mg to about 3 mg.
[8] The patch according to [6] or [7], which is adhered once a day or re-adhered once during 2 to 4 days.
[9] A blood concentration regulation type patch which comprises methyl 4-{[2-((1R,2R,3R)-3-hydroxy-2-{(1E,3S)-3-hydroxy-4-[3-(methoxymethyl)phenyl]but-1-enyl}-5-oxocyclopentyl)ethyl]sulfanyl}butanoate, a percutaneous permeation accelerator which is one or more substances selected from the group consisting of isopropyl myristate, crotamiton, oleic and oleyl alcohol and an adhesive which may contain an adhesion providing agent, a softening agent and/or an antioxidant, for use in preventing, treating and/or advance-suppressing a bone disease.

The percutaneous absorption preparation of the present invention can stably maintain blood concentration of its active form, 4-{[2-((1R,2R,3R)-3-hydroxy-2-{(1E,3S)-3-hydroxy-4-[3-(methoxymethyl)phenyl]but-1-enyl}-5-oxocyclopentyl)ethyl]sulfanyl}butanoic acid, and because of this, blood concentration of the active form can be continuously adjusted within a range of from a concentration which is effective for expressing its pharmacological activity to not exceeding a concentration which generates side effects.

### Best Mode for Carrying Out the Invention

The compound used in the present invention is a compound represented by a 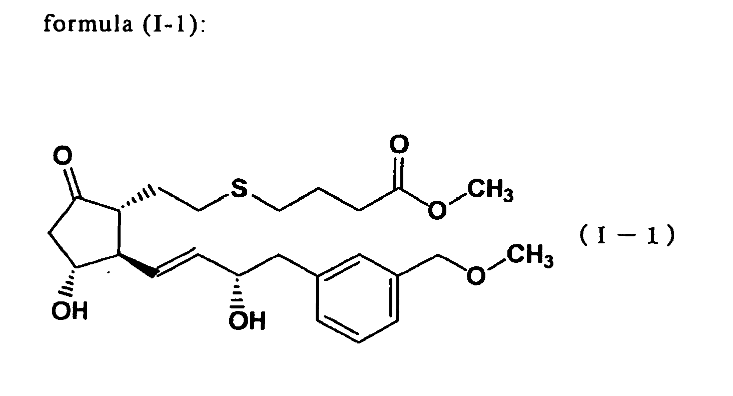 wherein all symbols have the same meaning as described above, namely methyl 4-{[2-((1R,2R,3R)-3-hydroxy-2-{(1E,3S)-3-hydroxy-4-[3-(methoxymethyl)phenyl]but-1-enyl}-5-oxocyclopentyl)ethyl]sulfanyl}butanoate (to be referred sometimes to as compound A hereinafter).

In this connection, their naming was carried out using a computer program, ACD/NAME (registered trademark, Advanced Chemistry Development, Inc.), which mechanically produces IUPAC name.

According to the present invention, the compound used in the present invention is not limited to substantially pure and single substances and may contain impurities (e.g., by-products derived from production process, solvents, materials, degradation products and the like) as long as they are within the pharmaceutically acceptable range. The impurity content acceptable as pharmaceuticals varies depending on the impurities contained therein. In the case of the compound A for example, it is preferable that the amount of each of the analogous substances as its degradation products is about 1.0% or less based on the entire amount, and total amount of the analogous substances is about 4.0% or less based on the entire amount.

The compound used in the present invention can be produced by using conventionally known methods, for example, the methods described in International Publication No. 00/003980, the method described in International Publication No. 03/009872, the method described in Comprehensive Organic Transformations: A Guide to Functional Group Preparations, Second Edition, (Richard C. La Roche John Wiley and Sons Inc, 1999) and the like, alone or by a combination of two or more thereof. Additionally, the thus produced compound can be purified by general purification means, for example, distillation under ordinal pressure or under a reduced pressure, a high performance liquid chromatography which uses silica gel or magnesium silicate or washing, recrystallization and the like.

In this connection, the PGE₁ which is used in the present specification is a conventionally known compound and is known as CAS registration number 745-65-3.

The compound whose blood concentration is regulated in the invention is the active form of the compounds of the present invention: 4-{[2-((1R,2R,3R)-3-hydroxy-2-{(1E,3S)-3-hydroxy-4-[3-(methoxymethyl)phenyl]but-1-enyl}-5-oxocyclopentyl)ethyl]sulfanyl}butanoic acid (to be referred sometimes to as the active main body of the present invention or compound B hereinafter).

The compound B is a compound represented by a formula (I-A): 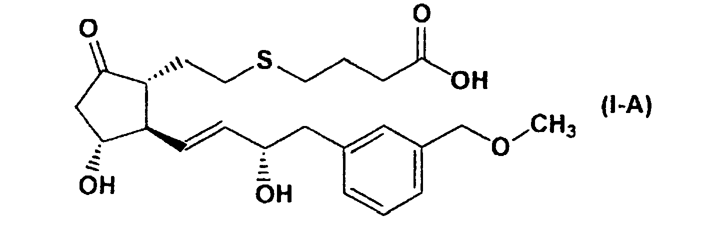 wherein all of the symbols have the same meanings as described above.

In the percutaneous absorption preparations of the present invention, the compound of the present invention is converted into the compound B at from the time of starting its skin permeation until the time when it reaches blood vessels, or the compound of the present invention is converted into compound B after reaching the blood vessels through its hydrolysis by an esterase or the like which present in the body. Accordingly, blood concentration of compound B can be appropriately regulated by the percutaneous absorption preparations of the present invention.

The blood concentration regulation type percutaneous absorption preparation of the present invention means a pharmaceutical preparation in which blood concentration of the active form of the present invention is maintained at the time of its administration within the range of from a concentration which is effective for expressing its pharmacological activity to not exceeding concentration which expresses the side effects. It also means a percutaneous absorption preparation in which when a side effect based on the pharmacological activity of the active form of the present invention, for example an influence upon the circulatory organs or the like, is generated, the side effect can be avoided by releasing said preparation due to quick reduction of blood concentration of the active form of the present invention. Additionally, it also means a percutaneous absorption preparation which contains the compound of the present invention in such an amount that its effective blood concentration is maintained and it does not cause skin blood vessel edema.

In the present specification, the skin blood vessel edema means a side effect or the like which is generally called swelling and caused by percutaneous absorption preparations, and also means the edema and the like described in Examples which are described later. Edema is a condition in which water is excessively accumulated in an extravascular subcutaneous tissue, for example, caused by the leaking of the body fluid in blood into outside of a blood vessel.

In the present specification; the base for external preparations means a pharmaceutical excipient to be used in external preparations.

As the base for external preparations of the percutaneous absorption preparations of the present invention, for example, one or more substances selected from the group consisting of an adhesive, an adhesion reinforcing agent, an adhesion providing agent, an amphipathic solubilizing agent, a suspending agent, a softening agent, an emulsifying agent, a buffer agent, an adhesive agent, a crosslinking agent, a skin irritation alleviating agent, an antioxidant and the like can be used.

In the present specification, examples of the adhesive or adhesion reinforcing agent include propylene glycol, di-t-butylhydroxytoluene, glycerol, glucono-δ-lactone, gelatin, dextrin, casein sodium, collodion, tragacanth, tragacanth powder, wheat starch, white petrolatum, petrolatum-lanolin alcohol mixture, hydrous lanolin, carnauba wax, starch syrup, aluminum magnesium silicate, soft silicic anhydride, sulfated castor oil potassium salt-alkyl benzene sulfonate mixture, benzyl acetate, talc, an acryl system adhesive (e.g., a (meth)acrylic acid-(meth)acrylic acid ester copolymer, an acrylic acid ester resin, an acryl system copolymer resin, an acrylic acid-acrylic acid octyl ester copolymer, an acrylic acid ester-vinyl acetate copolymer, a 2-ethylhexyl acrylate-vinyl pyrrolidone copolymer solution, a 2-ethylhexyl acrylate-2-ethylhexyl methacrylate-dodecyl methacrylate copolymer solution, a methyl acrylate-2-ethylhexyl acrylate copolymer resin emulsion, a methacrylate-n-butyl acrylate copolymer, an acrylic acid silk fibroin copolymer resin, an acrylic resin alkanolamine liquid, a starch acrylate, a polyacrylic acid, a polyacrylic acid aqueous solution, a sodium polyacrylate, a polyacrylic acid partial neutralization product, a carboxyvinyl polymer or the like), a rubber system adhesive (e.g., natural rubber, crude rubber, high molecular weight rubber, RSS No. I crude rubber, styrene isoprene rubber, styrene-butadiene rubber (SBR), cispolyisoprene rubber, a polyisobutylene (high molecular weight polyisobutylene, low molecular weight polyisobutylene or a mixture thereof with an optional ratio), high cispolyisoprene rubber, a styrene-isoprene-styrene block copolymer (SIS), a styrene-isobutylene-styrene block copolymer, a styrene-butadiene-styrene block copolymer (SBR), a styrene-butadiene-styrene block copolymer (SBS), acacia, powdered acacia, a rosin system resin (e.g., a maleylated rosin glycerol ester, rosin, a rosin derivative (e.g., an ultra-hypochromic rosin, an ultra-hypochromic rosin ester, an acid-modified ultra-hypochromic rosin, a rosin-containing diol, an ultra-hypochromic rosin metal salt or the like), a hydrogenated rosin glycerol ester, an ester gum or the like), dammar rubber or the like), a vinyl ester system adhesive (e.g., a methyl vinyl ether-maleic anhydride copolymer or the like), a vinyl ester system adhesive, a polyester system adhesive, a hydrophilic polymer (e.g., methyl cellulose, carboxymethylcellulose (CMC), carboxymethylcellulose sodium (CMCNa), hydroxyethylcellulose, hydroxypropylcellulose, hydroxypropylmethylcellulose (HPMC) (e.g., hydroxypropylmethylcellulose 2208, hydroxypropylmethylcellulose 2906, hydroxypropylmethylcellulose 2910 or the like), polyethylene glycol (e.g., macrogol 400, macrogol 6000 or the like), polyvinyl alcohol (PVA), polyvinyl pyrrolidone (PVP), sodium alginate, alginic acid propylene glycol ester, pectin, xanthan gum, xanthan gum, locust bean gum, guar gum, arabinogalactan, sodium hyaluronate or the like), animal and plant oils and fats (e.g., corn oil, castor oil or the like), polybutene, a maleylated rosin glycerol ester, an aliphatic hydrocarbon resin (Quintone, Escolez or the like), a terpene resin, a terpene-phenyl resin, a polyterpene system resin chroman-indene resin, a xylene resin and the like.

In the present specification, the adhesion providing agent is not particularly limited as long as it is a base which increases adhesiveness to the skin when mixed with an adhesive or adhesion reinforcing agent. Examples of it include a rosin system resin (e.g., a maleylated rosin glycerol ester, rosin, a rosin derivative (e.g., an ultra-hypochromic rosin, an ultra-hypochromic rosin ester, an acid-modified ultra-hypochromic rosin, a rosin-containing diol, an ultra-hypochromic rosin metal salt or the like), a hydrogenated rosin glycerol ester, an ester gum or the like), an alicyclic saturated hydrocarbon resin (e.g., Archon P100 or the like), an aliphatic hydrocarbon resin (e.g., Quintone B170 or the like), a hydrogenated terpene resin, a terpene resin, a polyterpene resin, a terpene-phenyl resin, a xylene resin, liquid rubber (e.g., polybutene, liquid polyisobutylene or the like), dit-butylhydroxytoluene, a polyisobutylene (high molecular weight polyisobutylene, low molecular weight polyisobutylene or a mixture thereof with an optional ratio) and the like.

In the present specification, the amphipathic solubilizing agent means a substance which has solubility in both of aqueous solvents and oil solvents and accelerates shifting of the compound of the present invention from its non-dissolving type to dissolving type accompanied by its percutaneous absorption. It is not particularly limited as long as it has such properties. Examples of the agent include N-methyl-2-pyrrolidone, lauric acid diethanol amide, triethyl citrate, dimethylimidazolidinone, a polyglycerol fatty acid ester, a polyoxyethylene alkyl formaldehyde condensate, a polyoxyethylene sterol-hydrogenated sterol, a polyoxyethylene glycol fatty acid ester, a polyoxyethylene lanolin, a sodium phosphate polyoxyethylene lauryl ether, a polyoxyethylene polyoxypropylene alkyl ether, a bees wax derivative, a polyoxyethylene alkyl amine-fatty acid amide, a polyoxyethylene alkyl ether phosphate-phosphoric acid salt, polyethylene glycol monooleate, triacetin, paniothenyl ethyl ether, ethylene glycol monobutyl ether, monoethanolamine, diethanolamine, diethylamine, isopropanolamine, diisopropanolamine, triethanolamine, N,N-dimethylacetamide, dimethyl sulfoxide, dodecyl sulfoxide, glycerol, geraniol, propylene glycol fatty acid ester, propylene carbonate, thioglycolic acid, propionic acid, methanesulfonic acid, glacial acetic acid, lactic acid, butyric acid, citric acid, hydrochloric acid, phosphoric acid, disodium hydrogenphosphate, sodium dihydrogenphosphate, potassium iodide, ichthammol, benzyl benzoate, nicotinic acid amide, nicotinic acid benzyl ester, soybean lecithin, hydrogenated soybean lecithin, D-mannitol, zinc sulfate, aluminum sulfate, sodium thiosulfate, middle chain fatty acid triglyceride, β-cyclodextrin, a polyhydric alcohol (e.g., polypropylene glycol 2000, ethoxy glycol, propylene glycol, 1,3-butylene glycol, polyethylene glycol 400, diethylene glycol, ethoxy diglycol, diisopropylene glycol or the like), liquid paraffins (e.g., light liquid paraffin, liquid paraffin and the like), animal and plant oils and fats (eg., rapeseed oil, soybean oil and the like), a higher fatty acid ester (e.g., isopropyl myristate, isopropyl palmitate, oleyl oleate or the like), a glycerol fatty acid ester (e.g., lipophilic glycerol monooleate or the like), a sorbitan fatty acid ester (e.g., sorbitan monooleate, sorbitan trioleate, sorbitan sesquioleate or the like), polyoxyethylene sorbitan fatty acid ester (e.g., polysorbate 20, polysorbate 60, polysorbate 80, polyoxyethylene sorbitan monolaurate or the like), a polyethylene glycol (e.g., lauromacrogol or the like), a polyoxyethylene polyoxypropylene glycol (e.g., polyoxyethylene (160) polyoxypropylene (30) glycol, polyoxyethylene (196) polyoxypropylene (67) glycol or the like), a polyoxyethylene alkyl phenyl ether (e.g., polyoxyethylene nonyl phenyl ether or the like), a higher alcohol (e.g., lauryl alcohol, isopropanol, isostearyl alcohol, octyldodecanol, oleyl alcohol or the like), a propylene glycol monocaprate (e.g., S-218 or the like), a fatty acid (e.g., capric acid, adipic acid, sebacic acid, myristic acid, oleic acid or the like), dibasic acid diesters (e.g., diethyl sebacate, diisopropyl sebacate, diisopropyl adipate and the like), polyoxyethylene castor oil/hydrogenated castor oils (e.g., monofatty acid polyoxyethylene hydrogenated castor oil, polyoxyethylene castor oil, polyoxyethylene hydrogenated castor oil, polyoxyethylene hydrogenated castor oil 10, polyoxyethylene hydrogenated castor oil 40, polyoxyethylene hydrogenated castor oil 50, polyoxyethylene hydrogenated castor oil 60 and the like), a terpene oil (e.g., peppermint oil, orange oil, turpentine oil, L-menthol, D-limonene, menthone, pinene, piperitone, terpinene, terpinolene, terpinol, carveol or the like), an organic solvent (e.g., methyl ethyl ketone, methyl isobutyl ketone, methanol, acetone, ethyl acetate, ethyl lactate, dimethyl ether, geraniol denatured alcohol, 8-acetylsucrose denatured alcohol, linalyl acetate denatured alcohol, phenylethyl alcohol denatured alcohol, benzyl alcohol, butanol, 2-butanol, polyvinyl alcohol, 2-amino-2-methyl-1-propanol, 2-amino-2-methyl-1-propanediol or the like) and the like.

In the present specification, the suspending agent is not particularly limited as long as it is a base in which the compound of the present invention can be suspended. Examples of the suspending agent includes ethanol, propylene glycol fatty acid ester, ethylene glycol monostearate, sorbitan sesquioleate, polyoxyethylene nonylphenyl ether, polyoxyethylene (160) polyoxypropylene (30) glycol, dioctyl sodium sulfosuccinate, dimethyl polysiloxane-silicon dioxide mixture, carboxyvinyl polymer, povidone, methylene-β-naphthalenesulfonate, sodium erythorbate, soybean lecithin, hydrogenated soybean lecithin, a middle chain fatty acid triglyceride, acacia, powdered acacia, xanthan gum, pectin, bentonite, sodium alginate, alginic acid propylene glycol ester, sodium chloride, benzalkonium chloride, kaolin, carrageenan, dried aluminum hydroxide gel, aluminum magnesium silicate, magnesium aluminometasilicate, potassium hydroxide, sodium hydroxide, aluminum stearate, phosphoric acid, waxes (e.g., bees wax, bleached bees wax, carnauba wax and the like), a polyhydric alcohol (e.g., stearyl alcohol, cetanol, ethylene glycol, propylene glycol, butanediol, triethylene glycol, polyethylene glycol (e.g., macrogol 200, macrogol 300, macrogol 400, macrogol 1500, macrogol 4000, macrogol 6000 or the like), cetomacrogol 1000, glycerol or the like), a higher fatty acid ester (e.g., hexyl laurate, butyl stearate, isopropyl palmitate, isopropyl myristate, octyldodecyl myristate, myristyl myristate, a glycerol fatty acid ester, glycerol monostearate, a sorbitan fatty acid ester or the like), a polyoxyethylene sorbitan fatty acid ester (e.g., polysorbate 20, polysorbate 60, polysorbate 80, polyoxyethylene sorbitan monolaurate or the like), liquid paraffins (e.g., light liquid paraffin, liquid paraffin and the like), animal and plant oils and fats (e.g., almond oil, camellia oil, persic oil, mink oil, safflower oil, coconut oil, eucalyptus oil, soybean oil, sesame oil, corn oil, rapeseed oil, sunflower oil, cotton seed oil, olive oil, castor oil, peanut oil, wheat germ oil, hardened oil, squalane, squalene and the like), polyoxyethylene castor oil/hydrogenated castor oils (e.g., polyoxyethylene hydrogenated castor oil, polyoxyethylene hydrogenated castor oil 60 and the like), celluloses (e.g., methylcellulose, hydroxypropylcellulose, carboxymethylcellulose, carboxymethylcellulose sodium, crystalline cellulose-carmellose sodium and the like), a sorbitan fatty acid ester (e.g., sorbitan monolaurate, sorbitan trioleate or the like) and the like.

In the present specification, the softening agent is not particularly limited as long as it is an agent which can plasticize and soften an adhesive and thereby keep its appropriate adhesiveness to the skin. For example, allantoin, propylene glycol, polybutene, crystalline cellulose, macrogol 1500, a middle chain fatty acid triglyceride, glycerol monostearate, isopropyl myristate, crude rubber, cetyl lactate, nonylic acid vanillyl amide, high-cis polyisoprene rubber, purified lanolin, animal and plant oils and fats (e.g., almond oil, olive oil, rapeseed oil, castor oil, cotton seed oil-soybean oil mixture, process oil, beef tallow, squalane, squalene and the like) and liquid paraffins (e.g., light liquid paraffins, liquid paraffin and the like).

In the present specification, the emulsifying agent is not particularly limited as long as it is a base which emulsifies the compound of the present invention. Examples of the emulsifying agent includes α-monoisostearyl glyceryl ether, ethylene glycol monostearate, a polyglycerol fatty, acid ester, diethanolamide laurate, a sucrose fatty acid ester, carboxyvinyl polymer, dioctyl sodium sulfosuccinate, potassium stearate, sodium stearate, carrageenan, potash soap, medical soap, an alkyl diaminoethylglycine hydrochloride liquid, a sulfated castor oil potassium-alkyl benzene sulfonate mixture, diisopropanolamine, triethanolamine, diethyl sebacate, ethanol, glycerol, a stearyl alcohol-polyoxyethylene stearyl ether mixture, a cetanol-polyoxyethylene cetyl ether mixed wax, a cetanol-polysorbate. 60 mixed wax, a cetanol-polyoxyethylene sorbitan monostearate mixed wax, a cetostearyl alcohol-cetostearyl sulfate sodium mixture, a pentaerythtyl citric acid higher fatty acid ester-bees wax-nonionic emulsifier mixture, dicetyl phosphate, sodium N-lauroyl-L-glutamate, benzoin tincture, a middle chain fatty acid triglyceride, sodium N-acyl-L-glutamate, benzalkonium chloride, potassium hydroxide, sodium hydroxide, talc, cetyl sulfate sodium, lauryl sulfate sodium, pectin, a coconut oil and fat fatty acid diethanolamide, a sorbitan fatty acid ester (e.g., sorbitan monooleate, sorbitan trioleate, sorbitan monolaurate, sorbitan sesquioleate, sorbitan monostearate, sorbitan tristearate, sorbitan monopalmitate or the like), a glycerol fatty acid ester (e.g., glycerol monooleate, lipophilic glycerol monooleate, glycerol monostearate, lipophilic glycerol monostearate, self emulsification type glycerol, monostearate, polyoxyethylene glyceryl triisostearate, a glycerol monooleate-glycerol dioleate-propylene glycol mixed emulsifier or the like), a polyoxyethylene glycerol fatty acid ester (e.g., polyoxyethylene glycerol monostearate or the like), a polyoxyethylene sorbitan fatty acid ester (e.g., polysorbate 20, polysorbate 40, polysorbate 60, polysorbate 65, polysorbate 80, polyoxyethylene sorbitan monolaurate, polyoxyethylene sorbitan monooleate, polyoxyethylene sorbitan trioleate, polyoxyethylene sorbitan tetraoleate or the like), a polyethylene glycol (e.g., macrogol 300, macrogol 400, macrogol 20000, cetomacrogol 1000 or the like), a polyoxyethylene alkyl ether (e.g., polyoxyethylene cetyl ether, polyoxyethylene nonylphenyl ether, polyoxyethylene octylphenyl ether, polyoxyethylene oleyl ether, polyoxyethylene cetostearyl ether, polyoxyethylene sorbitol bees wax, polyoxyethylene oleyl ether sodium phosphate, polyoxyethylene cetyl ether sodium phosphate, polyoxyethylene lanolin, lauromacrogol, polyoxyethylene behenyl ether, polyoxyethylene stearyl ether phosphate or the like), polyoxyethylene castor oil/hydrogenated castor oils (e.g., polyoxyethylene castor oil, polyoxyethylene hydrogenated castor oil, polyoxyethylene hydrogenated castor oil 5, polyoxyethylene hydrogenated castor oil 10, polyoxyethylene hydrogenated castor oil 20, polyoxyethylene hydrogenated castor oil 40, polyoxyethylene hydrogenated castor oil 50, polyoxyethylene hydrogenated castor oil 60 or the like), a polyoxyethylene polyoxypropylene glycol (e.g., polyoxyethylene (160) polyoxypropylene (30) glycol, polyoxyethylene (1) polyoxypropylene (1) cetyl ether, polyoxyethylene (10) polyoxypropylene (4) cetyl ether, polyoxyethylene (20) polyoxypropylene (4) cetyl ether, polyoxyethylene (20) polyoxypropylene (8) cetyl ether or the like), a polyhydric alcohol (e.g., propylene glycol, glycerol, cetanol, myristyl alcohol, octyldodecanol, oleyl alcohol, stearyl alcohol, cetostearyl alcohol, hydrogenated lanolin alcohol or the like), a polyethylene glycol fatty acid ester (e.g., polyethylene glycol monooleate, polyethylene glycol monostearate, polyethylene glycol distearate, polyethylene glycol monolaurate or the like), a propylene glycol fatty acid ester (e.g., propylene glycol monostearate or the like), a fatty acid ester (e.g., isopropyl myristate, octyldodecyl myristate or the like), celluloses (e.g., methyl cellulose, carboxymethylcellulose sodium, hydroxypropylcellulose or the like), an acrylic acid copolymer (e.g., methyl acrylate-2-ethylhexyl acrylate copolymer resin emulsion or the like), liquid paraffins (e.g., light liquid paraffin, liquid paraffin and the like), animal and plant oils and fats (e.g., mineral oil, cotton seed oil-soybean oil mixture, egg yolk oil, squalane, squalene and the like), lanolins (e.g., reduced lanolin, purified lanolin and the like), a lecithin derivative (e.g., egg yolk phospholipid, soybean lecithin, purified soybean lecithin, hydrogenated soybean phospholipid, partially hydrogenated soybean phospholipid or the like), a fatty acid (e.g., stearic acid or the like), polyoxyl stearates (e.g., polyoxyl stearate 40, polyoxyl stearate 45, polyoxyl stearate 55 and the like), waxes (e.g., bees wax, bleached bees wax, paraffin wax, spermaceti and the like) and the like.

In the present specification, examples of the buffer agent include benzalkonium chloride, diethanolamine, diisopropanolamine, monoethanolamine, triethanolamine, triethanolamine hydrochloride, triethanolamine phosphoric acid salt ester sodium liquid, chlorobutanol, glucose, rose oil, a organic acid (e.g., citric acid, citric anhydride, tartaric acid, succinic acid. DL-malic acid, fumaric acid, maleic acid, lactic acid, acetic acid; glacial acetic acid, benzoic acid, t-aminocaproic acid or the like), an inorganic acid (e.g., boric acid, borax, phosphoric acid or the like), an organic acid metal salt (e.g., sodium citrate, sodium lactate liquid, sodium acetate, sodium benzoate, sodium metaphosphate or the like), a metal hydroxide (e.g., disodium hydrogenphosphate, potassium dihydrogenphosphate, sodium dihydrogenphosphate, anhydrous disodium hydrogenphosphate, anhydrous sodium dihydrogenphosphate, sodium hydroxide or the like), primary, secondary or tertiary phosphate (e.g., sodium primary phosphate, sodium secondary phosphate, anhydrous sodium tertiary phosphate, sodium tertiary phosphate or the like), an amino acid or a salt of the amino acid (e.g., glycine, L-arginine or the like) and the like.

In the present specification, examples of the adhesive agent includes crosslinked or un-crosslinked acrylic copolymer, vinyl acetate adhesive, polyisobutylene (high molecular weight polyisobutylene, low molecular weight polyisobutylene or a mixture thereof with an optional ratio), neoprene, polybutadiene, polyisoprene, ethylene vinyl acetate copolymer, polysiloxane, polyacrylate, polyurethane, polyether block amide copolymer, styrene rubber block copolymer and the like.

In the present specification, examples of the crosslinking agent include an amino resin, a phenol resin, an epoxy resin, an alkyd resin, an isocyanate compound, a block isocyanate compound, a polyisocyanate compound (e.g., Nissetsu CK-101 or the like), an unsaturated polyester resin, an acrylic resin, a polyester resin, a urethane resin and the like.

In the present specification, examples of the skin irritation alleviating agent include glycerol, crotamiton and the like.

In the present specification, examples of the antioxidant include parabens (e.g., methyl paraben or the like), sorbic acid or salts thereof, di-t-butyl hydroxyanisole, di-t-butyl hydroxytoluene, nordihydroguaiaretic acid, guaiacol esters, 1,3-butylene glycol, sodium dehydrosulfate, a salt which forms a trivalent metal ion (e.g., aluminum chloride, alum, aluminum allantoinate or the like) and the like. In this connection, these can also be used as antiseptics.

In the patches of the present invention, a percutaneous permeation accelerator may be used further with the aforementioned base for external preparations.

In the present specification, the percutaneous permeation accelerator is not particularly limited as long as it is a compound which has the function to improve solubility and dispersibility of the compound of the present invention in the adhesive layer. According to the present invention, the percutaneous permeation accelerator may be used alone or in combination of two or more species. Examples of the percutaneous permeation accelerator include crotamiton, urea, ethanol, dimethyl sulfoxide, dimethyl acetamide, 2-pyrrolidone, N,N-diethyl-m-toluamide, N,N-dimethylformamide, N-methyl-2-pyrrolidone, calcium thioglycolate, oxazolidinone, a dioxolan derivative, a laurocaprum derivative, natural essential oil, lauryl diethanolamide, N-hydroxymethyl lactate, sorbitol, squalene, a triacetin (e.g., glyceryl triacetate or the like), a terpene oil (e.g., peppermint oil, orange oil, turpentine oil, L-menthol, D-limonene, menthone, pinene, piperitone, terpinene, terpinolene, terpinol, carveol or the like), a fatty acid ester (e.g., isopropyl myristate, glycerol monolaurate, glycerol monooleate, cetyl lactate, glycerol monolaurate, glycerol monooleate, propylene glycol monolaurate, propylene glycol, monooleate, sorbitan monolaurate, sorbitan monooleate, or the like), dibasic acid diesters (e.g., diethyl sebacate, diisopropyl adipate and the like), an azacyclo alkane (e.g., 1-dodecylazacycloheptane-2-one (Eizon), 1-(2-(decylthio)ethyl)azacyclopentan-2-one or the like), fatty acid or fatty acid alcohols (e.g., oleic acid, lauric acid, myristic acid, caproic acid, caprylic acid, capric acid, palmitic acid, isopalmitic acid, stearic acid, isostearic acid, arachic acid, behenic acid, lignoceric acid, myristoleic acid, palmitoleic acid, linoleic acid, linolenic acid, arachidonic acid, erucic acid, docosahexenoic acid, tetracosenoic acid, oleyl alcohol, isopropanol, lauryl alcohol and the like), a polyoxyethylene alkyl ether (e.g., polyoxyethylene (2) lauryl ether, polyoxyethylene (2) oleyl ether or the like), a middle chain fatty acid glyceride (e.g., caprylic acid monoglyceride or the like) and the like.

In addition to the aforementioned base for external preparations and percutaneous permeation accelerator, other additive agents generally used in the production of percutaneous preparations may be optionally mixed with the compound of the present invention. Examples of such additive agents include a solid filler (e.g., D-sorbitol, D-sorbitol liquid, sucrose or the like), a thickening agent (e.g., gelatin or the like), a powder filler (e.g., kaolin, bentonite, zinc oxide or the like), a surfactant (e.g., polyoxyethylene hydrogenated castor oil 20, polyoxyethylene hydrogenated castor oil 60, a polyoxyethylene sorbitan fatty acid ester (e.g., polysorbate 20, polysorbate 60, polysorbate 80, polyoxyethylene sorbitan monolaurate or the like), a polyoxyethylene fatty acid ester (e.g., polyoxyl stearate 40 or the like), a sorbitan fatty acid ester (e.g., sorbitan monooleate, sorbitan trioleate, sorbitan monolaurate, sorbitan sesquioleate, sorbitan monostearate or the like), self emulsification type glycerol monostearate, sucrose fatty acid ester, macrogol 400, lauromacrogol, sodium phosphate polyoxyethylene lauryl ether, phosphoric acid polyoxyethylene oleyl ether, polyoxyethylene nonylphenyl ether, polyoxyethylene octylphenyl ether, a polyoxyethylene polyoxypropylene glycol (e.g., polyoxyethylene (120) polyoxypropylene (40) glycol, polyoxyethylene (160) polyoxypropylene (30) glycol, polyoxyethylene (20) polyoxypropylene (20) glycol, or the like), polyoxyethylene polyoxypropylene decyl tetradecyl ether, alkyl aryl polyether alcohol, polyoxyethylene cetyl ether, polyoxyethylene oleylamine, polyoxyethylene sorbitol bees wax, lauric acid diethanolamide, stearyl alcohol, dibasic acid diesters (e.g., diethyl sebacate and the like), squalane, N-cocoyl-N-arginine ethyl ester-DL-pyrrolidone carboxylate, N-cocoyl-N-methylaminoethyl sulfonate sodium, cetanol, cetomacrogol 1000, sodium lauryl sulfate and the like), moisture keeping agents (e.g., alkaline earth metals (e.g., magnesium chloride), urea, glycerol, sodium hyaluronate and the like), flavors (e.g., peppermint oil, orange oil, Chamomillae Flos oil, spearmint oil, clove oil, turpentine oil, pine oil, peppermint water, Himalayan cedar oil, bergamot oil, eucalyptus oil, lavender oil, rose water, rose oil, Roman Chamomillae Flos oil, Peru balsam, D-camphor, DL-camphor, D-borneol, DL-borneol, DL-menthol, L-menthol, geraniol, methyl salicylate, cinnamaldehyde, piperonal and the like), a solvent (e.g., an organic solvent (e.g., methanol, ethanol, ethyl acetate, n-butyl acetate, isopropanol, diethyl ether, 2-ethyl-1,3-hexanediol, methanol denatured alcohol, methyl isobutyl ketone, methyl ethyl ketone or the like), hydrochloric acid, water, physiological saline, hydrous ethanol, an unsaturated fatty acid (e.g., oleic acid or the like), synthetic squalane, dipropylene glycol, ethylene glycol salicylate, petroleum benzine, trichloroethane, 8-acetylsucrose denatured alcohol and the like) and the like. These additive agents are formulated generally based on the ratios usually used in percutaneous absorption preparations. Additionally, these additive agents may be used alone or as a combination of two or more species.

Additionally, as the base for external preparations, for example, a water-soluble high molecular compound (e.g., a polyacrylic acid and a derivative thereof, a cellulose derivative, polyvinyl alcohol, gelatin, casein, polyethylene glycol, acacia, a methyl vinyl ether-maleic anhydride copolymer, a natural polysaccharide or the like), a tat-soluble high molecular compound (e.g., natural rubber, isoprene rubber, butyl rubber, a styrene-isobutylene-styrene block copolymer, a styrene butadiene copolymer, lanolin, vaseline, Plastibase, bees wax, spermaceti, solid paraffin or the like), a fatty acid and a derivative thereof (e.g., a fatty acid having from 3 to 40 carbon atoms, a fatty acid ester thereof or a fatty acid alkali metal salt thereof or the like), animal and plant oils and fats (e.g., olive oil, peppermint oil, soybean oil, cotton seed oil, corn oil, eucalyptus oil, castor oil, sesame oil, almond oil, camellia oil, persic oil, mink oil, safflower oil, coconut oil and the like), alcohols (alcohols which have 1 to 40 carbon atoms and also have 1 to 70 hydroxyl in the molecule, such as ethanol, glycerol, propylene glycol, polyethylene glycol, octanediol, butanediol, D-sorbitol, benzyl alcohol and the like), a terpene oil (e.g., peppermint oil, orange oil, turpentine oil, L-menthol, D-limonene, menthone, pinene, piperitone, terpinene, terpinolene, terpinol, carveol or the like), a surfactant (e.g., a nonionic surfactant (e.g., polyoxyethylene sorbitan monooleate or the like), an anionic surfactant, a cationic surfactant or the like) and water can also be used. These may be used alone or as a combination of two or more species, or in combination with the aforementioned base for external preparations.

Additionally, in order to obtain good skin transfer of the agent of the percutaneous absorption preparations, particularly patches (to be referred sometimes to as the patches of the present invention hereinafter), it is necessary to fix the patches to the skin surface securely, regardless of the applying region. However, when their adhesiveness to the skin is too large, for example, in peeling and removing the patches atter their use, there is a possibility of causing skin irritation such as generation of keratin separation due to its physical stimulus. Accordingly, there is an oil gel adhesion technique as a method for improving adhesiveness of patches to the skin and thereby suppressing physical skin irritation. An easily separable adsorbent, an adhesive which forms an adhesive layer for the skin face fixing and a liquid component are used in the oil gel adhesion technique. Examples of the easily separable adsorbent include a crosslink type acrylic adhesive polymer, an ethylene-acetic acid-vinyl copolymer, or an ethylene-ethyl acrylate copolymer or the like. Examples of the adhesive which forms an adhesive layer for the skin face fixing include those in which a natural rubber system adhesive, a synthetic rubber system adhesive, an acrylic system adhesive, a vinyl ether system adhesive and the like are used as the base and their adhesiveness is adjusted by mixing with a softening agent, an additive agent and the like. Examples of the softening agent include mineral oil and the like. The liquid component is blended for example for adjusting the adhesiveness, and the aforementioned softening agent and a fatty acid ester and the like, are used.

The percutaneous absorption preparations of the present invention include ointments (e.g., lotions, creams, gels or the like), plastered cream preparations (e.g., patches, fomentations or the like) and the like. More preferable preparations are plastered cream preparations, and further preferable preparations are patches. Examples of the patches include plaster preparations (e.g., matrix (adhesive single layer) type patches, reservoir type patches or the like), cataplasms and the like. Additionally, drug dispersion type matrix type patches, drug dissolution type matrix type patches and the like are included in the matrix type patches. According to the present invention, plaster preparations, particularly matrix type patches, are preferable. The matrix type patches are constituted from a patch structure formation consisting, of a "drug-containing adhesive layer" having adhesiveness, which is formed by combining the compound of the present invention with an optional base selected from the aforementioned bases for external preparations, a "backing layer" and a "separator" which protects the drug-containing adhesive layer A non-dissolving type compound of the present invention or a mixed type compound of the present invention of dissolving type and non-dissolving type is contained in the drug-containing adhesive layer of the drug dispersion type matrix type patches, and a dissolution type compound of the present invention is contained in the drug-containing adhesive layer of the drug dissolution type matrix type patches. According to the present invention, the drug dispersion type matrix type patches are most preferable. Additionally, in order to withstand long-term application to the skin and prevent adhesive transfer to the skin surface at the time of separation removal, thickness of the drug-containing adhesive layer is preferably from about 10 µm to about 500 µm, more preferably from about 10 µm to about 200 µm, particularly preferably from about 20 µm to about 200 µm in the case of the plaster preparations. In the case of the cataplasms, it is preferably from about 300 µm to about 2000 µm, more preferably from about 500 µm to about 1500 µm.

It is preferable that the plaster preparations of the present invention have a tensile tester-aided tensile strength of from about I N to about 12 N, and is more preferable from about 1 N to about 5 N, according to the second adhesion test method (JIS-Z 0237-1991 Testing Methods of Pressure Sensitive Tapes and Sheets "180 degree peeling method") based on the self-imposed standard on first-aid plasters.

The drug-containing adhesive layer of the patches of the present invention contains the compound of the present invention and an adhesive, and contains one or more substances optionally selected from an amphipathic solubilizing agent, a suspending agent, a softening agent, an emulsifying agent, a buffer agent, an adhesion reinforcing agent, adhesion providing agent, a crosslinking agent, a skin irritation alleviating agent, an antioxidant and a percutaneous permeation accelerator. The preparation which comprises the compound of the present invention and an adhesive, and one or more substances optionally selected from an amphipathic solubilizing agent, an adhesion providing agent, a crosslinking agent, a skin irritation alleviating agent and a percutaneous permeation accelerator is preferable and the preparation which comprises adhesion providing agent, a softening agent and an antioxidant and further optionally contain a percutaneous permeation accelerator is more preferable. In this connection, the same substances as described in the foregoing can be used as the adhesive, amphipathic solubilizing agent, suspending agent, softening agent, antioxidant, emulsifying agent, buffer agent, adhesion reinforcing agent, adhesion providing agent, crosslinking agent, skin irritation alleviating agent and percutaneous permeation accelerator.

As the adhesive and/or adhesion reinforcing agent in the patches of the present invention, for example, a styrene-isoprene-styrene block copolymer (SIS), a polyisobutylene (high molecular weight polyisobutylene, low molecular weight polyisobutylene or a mixture thereof with an optional ratio), an acrylic acid ester resin, an acrylic system copolymer resin and the like are preferable. Although the styrene-isoprene-styrene block copolymer is not particularly limited, SIS-5009 and SIS-5229 (both trade names) and the like manufactured by JSR Corporation are preferably used. Although the acrylic acid ester resin is not particularly limited, Nissetsu PE-300 (trade name) and the like manufactured by Nippon Carbide Industries are preferably used. Although the acrylic system copolymer resin is not particularly limited, Nikasol TS-620 (trade name) and the like manufactured by Nippon Carbide Industries are preferably used.

As the adhesion providing agent in the patches of the present invention, for example, a rosin system resin (e.g., a maleylated rosin glycerol ester, rosin, a rosin derivative (e.g., an ultra-hypochromic rosin, an ultra-hypochromic rosin ester, an acid-modified ultra-hypochromic rosin, a rosin-containing diol, an ultra-hypochromic rosin metal salt or the like), a hydrogenated rosin glycerol ester, an ester gum or the like) and an alicyclic saturated hydrocarbon resin or the like are preferable. As the rosin derivative, an ultra-hypochromic rosin ester KE-311 (trade name) or the like manufactured by Arakawa Chemical Industries is preferably used. Although the alicyclic saturated hydrocarbon resin is not particularly limited, Alcon PE-100 (trade name) manufactured by Arakawa Chemical Industries is preferably used.

As the amphipathic solubilizing agent in the patches of the present invention, for example, one or two or more substances selected from a higher fatty acid ester (e.g., isopropyl myristate, isopropyl palmitate, oleyl oleate or the like), a higher alcohol (e.g., lauryl alcohol, isopropanol, isostearyl alcohol, octyl dodecanol, oleyl alcohol or the like), a fatty acid (e.g., capric acid, adipic acid, sebacic acid, myristic acid, oleic acid or the like), dibasic acid diesters (e.g., diethyl sebacate, isopropyl sebacate, diisopropyl adipate and the like), liquid paraffins (e.g., liquid paraffin, light liquid paraffin and the like), triacetin, benzyl alcohol and/or L-menthol are preferable, and 1 to 3 substances selected from liquid paraffin, light liquid paraffin, isopropyl myristate and oleyl alcohol are particularly preferable. Although the light liquid paraffin is not particularly limited, No. 70-S (trade name) manufactured by Sanko Chemical Industry Co., Ltd. is preferable.

As the crosslinking agent in the patches of the present invention, for example, a polyisocyanate system compound is preferable. As the polyisocyanate system compound, for example, Nissetsu CK-101 (trade name) and the like manufactured by Nippon Carbide Industries are preferable.

As the skin irritation alleviating agent in the patches of the present invention, for example, crotamiton or the like is preferable.

As the percutaneous permeation accelerator in the patches of the present invention, for example, fatty acid or aliphatic alcohols (e.g., oleic acid, lauric acid, myristic acid, oleyl alcohol, isopropanol, lauryl alcohol and the like), a fatty acid ester (e.g., glycerol monolaurate, glycerol monooleate, cetyl lactate, propylene glycol monolaurate, propylene glycol monooleate, sorbitan monolaurate, sorbitan monooleate, isopropyl myristate or the like), a terpene oil (e.g., peppermint oil, orange oil, turpentine oil, L-menthol, D-limonene, menthone, pinene, piperitone, terpinene, terpinolene, terpinol, carveol or the like), crotamiton and the like are preferable, and oleic acid, isopropyl myristate, D-menthol, crotamiton, oleyl alcohol and the like are particularly preferable.

Ratios of the aforementioned respective bases for external preparations, based on the drug-containing adhesive layer mass, in the patches of the present invention are not particularly limited as long as they are such ratios wherein the materials can be prepared generally as patches and the object of the present invention can be achieved.

For example, the base for external preparations in the patches of the present invention preferably contain a combination of one base selected as an adhesive, adhesion reinforcing agent and/or adhesion providing agent, from (1) a combination of a syrene-isoprene-styrene block copolymer and a rosin derivative, (2) a combination of a styrene-isoprene-styrene block copolymer and an alicyclic saturated hydrocarbon resin, (3) a combination of a styrene-isoprene-styrene block copolymer, polyisobutylene and a rosin derivative, (4) an acrylic acid ester resin, (5) an acrylic system copolymer resin and (6) a combination of a styrene-isoprene-styrene block copolymer, a rosin derivative, polyisobutylene and an alicyclic saturated hydrocarbon resin, with one or more bases selected as an amphipathic solubilizing agent, skin irritation alleviating agent and/or softening agent, from (a) L-menthol, (b) light liquid paraffin and (c) liquid paraffin. The base may be further combined with optional one or more bases selected as a percutaneous permeation accelerator, from the group consisting of (i) isopropyl myristate, (ii) crotamiton, (iii) oleic acid and (iv) oleyl alcohol, or may be further combined with a polyisocyanate compound as a crosslinking agent.

For example, the base for external preparations in the patches of the present invention more preferably contain a base selected as an adhesive and an adhesion providing agent, from (1) a combination of a styrene-isoprene-styrene block copolymer and a rosin derivative, (2) a combination of a styrene-isoprene-styrene block copolymer and an alicyclic saturated hydrocarbon resin and (3) a combination of a styrene-isoprene-styrene block copolymer, a rosin derivative, an alicyclic saturated hydrocarbon resin, di-t-butylhydroxytoluene and polyisobutylene, and one or more bases selected as a softening agent, from (a) light liquid paraffin and (b) liquid paraffin. The base may be used by further combining with optional one or more bases selected as a percutaneous permeation accelerator, from the group consisting of (i) isopropyl myristate, (ii) crotamiton and (iii) oleyl alcohol.

Example, the base for external preparations in the patches of the present invention includes further preferably a base selected as an adhesive and an adhesion providing agent, from the group consisting of a styrene-isoprene-styrene block copolymer, a rosin system resin, an alicyclic saturated hydrocarbon resin and polyisobutylene; a base which is a combination of one or more substances selected from di-t-butylhydroxytoluene as an antioxidant and liquid paraffin and light liquid paraffin as softening agents; a base in which one or more substances selected from isopropyl myristate and oleyl alcohol, as a percutaneous permeation accelerator and the like.

Examples of a particularly preferred combination of the compound of the present invention with a base for external preparations in the patches of the present invention include patches which comprise methyl 4-{[2-((1R,2R,3R)-3-hydroxy-2-{(1E,3S)-3-hydroxy-4-[3-(methoxymethyl)phenyl]but-1-enyl}-5-oxocyclopentyl)ethyl]sulfanyl}butanoate, a base comprising a combination of a styrene-isoprene-styrene block copolymer, a rosin system resin, an alicyclic saturated hydrocarbon resin, high molecular weight polyisobutylene and low molecular weight polyisobutylene, as an adhesive, adhesion reinforcing agent and/or adhesion providing agent, and a base comprising a combination of di-t-butylhydroxytoluene as an antioxidant and liquid paraffin as softening agent, and the like. Examples of a percutaneous permeation accelerator to be further used optionally include oleyl alcohol and the like.

Although the backing laver which constitutes the structure formation of the matrix type patches of the present invention is not particularly limited, those which have such a degree of flexibility wherein a significant sense of incongruity is not generated when applied to the skin surface are preferable For example, single layer films consisting of plastic film (e.g., polyethylene, polyethylene terephthalate, polyurethane, polyethylene, ethylene vinyl acetate, polypropylene, polyester, polyvinyl acetate, ethylene-vinyl acetate copolymer or the like), metal foil (e.g., aluminum foil or the like), non-woven fabric, cotton, woven fabric, knitting and the like and laminated films thereof can be used.

The separator is not particularly limited with as long as it can be easily peeled off when the patches of the present invention are used and can keep the drug-containing adhesive layer before covering with the separator. Specifically, for example, paper to which a fluorine resin treatment was applied, plastic films (e.g., polyethylene, polyethylene terephthalate, polyurethane, polyethylene, ethylene vinyl acetate, polypropylene, polyester, polyvinyl acetate, ethylene-vinyl acetate copolymer and the like) and the like can be used.

Preferable administration method of the patches of the present invention is a method in which they are re-plastered I to 4 times a day or once during 2 to 7 days, or plastered in response to the necessary situation such as before going to bed. More preferable method is a method in which they are re-plastered once a day or once during 2 to 4 days, or plastered in response to the necessary situation such as before going to bed.

Although the applying region of the patches of the present invention is not particularly limited as long as it is an applicable position, for example, it is the upper arm, the belly, the chest, the waist and back, the hips, the leg (e.g., the inner side of the thigh, the calf or the like) or the like, and 1 or 2 to 4 sheets are applied to the same or different regions described above. When two or more sheets are plastered, although the positions are not particularly limited, it is preferable to apply them to symmetrical positions. Additionally, in order to avoid skin irritation, it is not preferable to apply them continuously to the same position.

The plastering area of the patches of the present invention means an area where the compound of the present invention is applied to the patch base. The patching area is preferably from about 1 cm² to about 200 cm², more preferably from about 1 cm² to about 50 cm², particularly preferably from about 1 cm² to about 10 cm². In the case of plasters, it is preferably from about 1 cm² to about 200 cm², more preferably from about 1 cm² to about 50 cm², particularly preferably from about 1 cm² to about 10 cm². In the case of cataplasms, it is preferably from about 10 cm² to about 200 cm², more preferably from about 10 cm² to about 100 cm² Additionally, although it may have any shape, it is preferably square, rectangle or those having rounded four corners, circle, oval or the like shape which is generally used as plasters and cataplasms.

Although obvious to those skilled in the art, blood concentration of the active form of the present invention in patients can be expressed by a value (Css) obtained by dividing *in vitro* permeation rate by total body clearance.

Regarding the amount of the compound of the present invention to be formulated in the patches of the present invention, it varies depending on the administration period of time, the bases for external preparations to be used, their blending amounts and the like. As range of from a concentration which is effective blood concentration for achieving prevention, treatment and/or advance suppression of diseases to a concentration which does not express side effects in percutaneously administering it to mammals including human, it is not particularly limited as long as it is a concentration that achieves the object. Specifically, it is not particularly limited as long as blood concentration of the active form of the present invention in the human body is within the range of preferably from about 0.01 pg/ml to about 10 pg/ml, more preferably from about 0.2 pg/ml to about 4 pg/ml. Namely, its maximum blood concentration at the time of plastering is preferably about 4 pg/ml, more preferably about 2 pg/ml. On the other hand, as its blood concentration at the time of plastering, it is preferable that it is not lower than about 0.1 pg/ml, it is more preferable that it is not lower than about 0.2 pg/ml, for a period of 6 hours or more. For the purpose of achieving such a blood concentration, for example, the compound of the present invention is blended in an amount of preferably from about 0.001 mg to about 20 mg, more preferably from about 0.005 mg to about 5 mg, further preferably from about 0.01 mg to about 3 mg, per one preparation or one dose. Additionally, amount of the compound of the present invention is preferably from about 0.002% by mass to about 10% by mass, more preferably from about 0.02% by mass to about 5% by mass, further preferably from about 0.1% by mass to about 3% by mass, based on the entire drug-containing adhesive layer.

Blending amount of the compound of the present invention per plastering area is preferably from about 1 µg/cm² to about 2 mg/cm², more preferably from about 1 µg/cm² to about 200 µg/cm², further preferably from about 10 µg/cm² to about 100 µg/cm².

When methyl 4-{[2-((1R,2R,3R)-3-hydroxy-2-{(1E,3S)-3-hydroxy-4-[3-(methoxymethyl)phenyl]but-1-enyl}-5-oxocyclopentyl)ethyl]sulfanyl}butanoate (compound A) is used as the active ingredient, the following composition is preferable for the base for external preparations.

As the adhesive, a styrene-isoprene-styrene block copolymer, an acrylic acid ester resin and an acrylic acid copolymer resin are preferable, and a styrene-isoprene-styrene block copolymer is more preferable. When a styrene-isoprene-styrene block copolymer is used, its blending amount is preferably from about 10 parts by mass to about 200 parts by mass, more preferably from about 20 parts by mass to about 100 parts by mass, based on 1 part by mass of compound A. When an acrylic acid ester resin and/or an acrylic acid copolymer resin is used, its blending amount is preferably from about 50 parts by mass to about 1000 parts by mass, more preferably from about 100 parts by mass to about 500 parts by mass, based on 1 part by mass of compound A.

The adhesion providing agent may be used alone or as a combination of two or more substances. As the adhesion providing agent, one or more of bases selected from a rosin system resin, an alicyclic saturated hydrocarbon resin and a polyisobutylene. When a rosin resin is used, its blending amount is preferably from about 1 part by mass to about 100 parts by mass, more preferably from about 3 parts by mass to about 10 parts by mass, based on I part by mass of compound A. When an alicyclic saturated hydrocarbon resin is used, its blending amount is preferably from about 20 parts by mass to about 300 parts by mass, more preferably from about 50 parts by mass to about 100 parts by mass, based on 1 part by mass of compound A. When a polyisobutylene is used, each of the high molecular weight isobutylene and low molecular weight isobutylene is preferable, and it is preferable also to use both of them as a mixture. Regarding their blending ratio, it is preferably from about 1 part by mass to about 100 parts by mass of high molecular weight polyisobutylene and/or from about 5 parts by mass to about 200 parts by mass of low molecular weight polyisobutylene, more preferably from about 3 parts by mass to about 50 parts by mass of high molecular weight polyisobutylene and from about 10 parts by mass to about 75 parts by mass of low molecular weight polyisobutylene, based on 1 part of compound A.

As the softening agent according to the patches of the present invention, liquid paraffins are preferable, and light liquid paraffin and liquid paraffin are more preferable. As the blending ratio of liquid paraffin, from about 30 parts by mass to about 500 parts by mass based on 1 part by mass of compound A is preferable.

According to the patches of the present invention, a percutaneous permeation accelerator may be used together with the base for external preparations of the present invention if necessary. As the percutaneous permeation accelerator, at least one base selected from the group consisting of isopropyl myristate, crotamiton, oleic acid and oleyl alcohol is preferable, and isopropyl myristate and oleyl alcohol are more preferable. As the blending ratio of isopropyl myristate, it is preferably from about 0.5 pan by mass to about 20 parts by mass, more preferably from about 1 part by mass to about 10 parts by mass, based on 1 part by mass of compound A. As the blending ratio of oleyl alcohol, it is preferably from about 1 part by mass to about 50 parts by mass, more preferably from about 1 part by mass to about 30 parts by mass, based on 1 part by mass of compound A.

In the pharmaceutical preparations of the present invention, di-t-butylhydroxytoluene may be used as an antioxidant, and its blending ratio is preferably from about 0.1 part by mass to about 3 parts by mass, more preferable from about 0.15 part by mass to about 2 parts by mass, based on 1 part by mass of compound A.

According to the percutaneous absorption preparations of the present invention, at least one substance selected from the group consisting of a styrene-isoprene-styrene block copolymer, an acrylic acid ester resin and an acrylic acid copolymer resin is preferable as the adhesive, and their blending amount is preferably from about 0.1 mg to about 100 mg, more preferably from about 1 mg to about 50 mg, per one preparation or one dose.

Liquid paraffins (light liquid paraffin or liquid paraffin) may be added as a softening agent to the patches of the present invention if necessary, and its blending amount is preferably from about 3 mg to about 400 mg, more preferably from about 10 mg to about 100 mg, per one preparation or one dose.

When polyisobutylene is used as adhesion providing agent in the patches of the present invention, it contains preferably from about 0.3 mg to about 50 mg of high molecular weight polyisobutylene and/or from about 1 mg to about 70 mg of low molecular weight polyisobutylene, more preferably from about 1 mg to about 10 mg of high molecular weight polyisobutylene and from about 2 mg to about 20 mg of low molecular weight polyisobutylene, per one preparation or one dose.

When a rosin system resin is used as the adhesion providing agent in the patches of the present invention, it is contained in an amount of preferably from about 0.1 mg to about 70 mg, more preferably from about 0.3 mg to about 50 mg, per one preparation or one dose. When an alicyclic saturated hydrocarbon resin is used as the adhesion providing agent, it is contained in an amount of preferably from about 1 mg to about 100 mg, more preferably from about 5 mg to about 50 mg, per one preparation or one dose.

When di-t-butylhydroxytoluene is used as the antioxidant in the patches of the present invention, it is contained in an amount of preferably from about 0.03 mg to about 0.9 mg, more preferably from about 0.1 mg to about 0.5 mg, per one preparation or one dose.

The compound of the present invention may be blended as any one of dissolving type, non-dissolving type or a mixed type of the dissolving type and non-dissolving type. The type is not particularly limited as long as it can maintain its stability and safety; the compound of the present invention is efficiently and continuously permeated in skin; and it can be converted into the active form of the present invention and thereby can adjust blood concentration of the active form of the present invention. Specifically, the dissolving type compound of the present invention alone is blended or mixed type compound of the present invention of the dissolving type and non-dissolving type is blended.

Generally, in the case of patches, while a drug under a non-dissolving type state is not concerned in the percutaneous absorption, when the content of dissolving type drug in the drug-containing adhesive layer becomes large, amount of the drug which is quickly absorbed also becomes large. Therefore, it becomes possible to keep the drug effect for a prolonged period of time. In other words, duration of the pharmacological activity is restricted by the saturated solubility of the drug in the base for external preparations. However, in the case of a base for external preparations having low solubility of the drug, duration and continuation of stable effective blood concentration of the drug sometimes become insufficient. In order to obtain persistency of proper drug elect, its dose is increased by a means such as use of a base for external preparations having further high solubility, thickening of the drug-containing adhesive layer in which the drug was dissolved, increase of percentage content of the drug or enlargement of the area of the drug-containing adhesive layer contacting to the skin. However, these methods also have problems in terms of a sense of incongruity, adhesiveness, skin irritation, economy and the like.

Concentration of the dissolving type drug in the drug-containing adhesive layer of patches directly exerts influence upon percutaneous absorption rate and is reduced by its absorption into the skin. Since excess drug exceeding its saturated solubility in the drug-containing adhesive layer to be used is dispersed in the drug-containing adhesive layer as a non-dissolving type drug, amount of the dissolving type contained in the drug-containing adhesive layer is determined by the base for external preparations to be used.

On the other hand, the non-dissolving type drug has a function to supplement the decreased dissolving type drug due to skin-absorption of the drug dissolved in the drug-containing adhesive layer of the patches, by providing it into the drug-containing adhesive layer. As a result, high percutaneous absorption rate is kept for a long time and effective blood concentration is maintained for a long of time.

Accordingly, as the patches of the present invention, matrix type patches having percutaneous absorption ability, stable blood concentration pattern and proper drug effect persistency can be provided by selecting a drug-containing adhesive layer having high saturated solubility to prepare patches containing the dissolving type compound of the present invention or patches containing mixed type compound of the present invention of the dissolving type and non-dissolving type.

In the patches of the present invention, the dissolving type compound of the present invention means that the compound of the present invention is present in the drug-containing adhesive layer under a completely dissolved state, namely that the compound of the present invention is not observed in the drug-containing adhesive layer with the naked eye or under an optical microscope, wherein the drug-containing adhesive layer is uniform. Additionally, it is preferable that the drug does not precipitate at a high concentration so that the drug can be maintained under a completely dissolved state in the drug-containing adhesive layer In order to maintain high concentration of the compound of the present invention as the dissolving type, an additive agent may be further used. The additive agent is not particularly limited as long as it has superior compatibility with the adhesive; can dissolve the compound of the present invention sufficiently; and the adhesive and additive agents are not separated periodically. By this, effective blood concentration can be maintained for a longtime due to excellent percutaneous absorption rate in the initial stage of administration.

In the present invention, the mixed type compound of the present invention of the dissolving type and non-dissolving type means that the compound of the present invention is present in the drug-containing adhesive layer in both dissolved state and non-dissolved state. In order to supplement for the reduction of the compound of the present invention in the drug-containing adhesive layer caused by quick absorption of its dissolving type, re-dissolution of the non-dissolving type compound of the present invention quickly occurs and the drug effect persistency is maintained by the absorption of the dissolved compound of the present invention. Preferable ratio of the disappearing rate of the non-dissolving type compound of the present invention to the disappearing rate of all compounds of the present invention in the drug-containing adhesive layer is about 0.1 or more. When the ratio of disappearing rates is low, re-dissolution of the non-dissolving type drug becomes insufficient for the reduction of dissolving type drug, so that persistency of the drug effect is not always good.

The patches of the present invention can be produced by the general methods shown in the following. The solution or dispersion to be used in the drug-containing adhesive layer is prepared, for example, by (1) a solvent method, (2) a heating method (hot melt method) or (3) a calender method. The solvent method is a method in which the compound and base for external preparations are dissolved in a solvent such as hexane, rubber gasoline, ethyl acetate, xylene, chloroform or the like and the solvent is evaporated after spreading the solution. After the spreading, it is dried preferably at about 20°C to about 60°C for about 30 seconds to about 24 hours. The heating method is a method in which the compound and base for external preparations are dissolved and mixed at a high temperature, and then cooled after spreading. The calender method is a method in which the compound and base for external preparations are mixed using a mixer and spread using a calender roller. Additionally, when they have relatively low viscosities, the compound and base for external preparations are mixed and spread using a general mixer. The patches can be produced by coating and drying; on a separator for protection use, the solution or dispersion obtained in this manner to be used in the drug-containing adhesive layer; thereby forming the drug-containing adhesive layer on the separator, and subsequently carrying out adhesion of a backing layer to said drug-containing adhesive layer As the production method of the patches of the present invention, the solvent method is preferable. Additionally, the production method of the patches of the present invention is not limited to the above methods, and they may be produced by other efficient methods.

### Toxicity

Since toxicity of the compound of the present invention is sufficiently low and the percutaneous absorption preparations of the present invention can suppress various side effects of the active form of the present invention to sufficiently low levels, they can be used safely as pharmaceutical preparations.

### Application to pharmaceutical preparations

Since the active form of the present invention has EP4 agonist activity, it can be used as percutaneous absorption preparations containing the compound of the present invention, for the purpose of preventing, treating and/or advance-suppressing diseases such as immune diseases (e.g., autoimmune diseases (e.g., amyotrophic lateral sclerosis (ALS), multiple sclerosis, Sjogren syndrome, articular rheumatism, systemic lupus erythematosus (SLE) and the like), rejection reaction after organ transplantation and the like), asthma, osteodystrophy, nerve cell death, pulmonary injury, hepatopathy, acute hepatitis, glomerulonephritis, renal insufficiency, hypertension, myocardial ischemia, systemic inflammatory response syndrome, pain due to burn, sepsis, hemophagocytosis syndrome, macrophage activation syndrome, Still disease, Kawasaki disease, burn, systemic granuloma, ulcerative colitis, Crohn disease, hypercytokinemia during dialysis, multiple organ failure, shock, dyssomnia, platelet agglutination, gastrointestinal ulcer (e.g., gastric ulcer, duodenal ulcer and the like), stomatitis, boldness, alopecia, bone disease and the like in mammals (e.g., human and non-human animals such as monkey, sheep, cow, horse, dog, cat, rabbit, rat, mouse and the like). In this connection, examples of the bone disease include (1) bone fracture, bone metastasis of cancer, hypercalcemia, Paget disease, bone defect (alveolar bone defect, mandibule defect, childhood idiopathic bone defect and the like), osteonecrosis, (2) primary osteoporosis (e.g., primary osteoporosis accompanied by aging, primary osteoporosis accompanied by menopause, primary osteoporosis accompanied by oophorectomy, vertebral body fracture and the like), (3) secondary osteoporosis (e.g., glucocorticoid-induced osteoporosis, thyroid function induced osteoporosis, fixation induced osteoporosis, heparin induced osteoporosis, immunosuppression induced osteoporosis, osteoporosis due to renal insufficiency, inflammatory osteoporosis, osteoporosis accompanied by Cushing syndrome, rheumatic osteoporosis and the like) and the like. Furthermore, the active form of the present invention can be used as percutaneous absorption preparations comprising the compound of the present invention, for the purpose of accelerating and healing-accelerating osteogenesis after bone operation (e.g., osteogenesis after bone fracture, osteogenesis after bone transplantation, osteogenesis after artificial joint replacement, osteogenesis after spine fusion, osteogenesis after other bone reconstructive operations and the like). Additionally, the compound of the present invention can also be used in bone transplantation, substitution therapy and the like.

Among the aforementioned various diseases, bone diseases are the most preferable diseases as application of the active form of the present invention, and bone fracture and vertebral body fracture are particularly preferable Additionally, it is also preferable to use the active form of the present invention as percutaneous absorption preparations comprising the compound of the present invention with the aim of accelerating and heating-accelerating osteogenesis after bone operation (e.g., osteogenesis after bone fracture, osteogenesis after bone transplantation, osteogenesis after artificial joint replacement, osteogenesis after spine fusion, osteogenesis after other bone reconstructive operations and the like).

With the object of the prevention, treatment and/or advance-suppression and the like of the aforementioned diseases, the compound of the present invention is prepared into an form which can be easily administered to patients by combining the aforementioned bases for external preparations, namely into percutaneous absorption preparations, and then administered into the living body.

The percutaneous absorption preparations prepared using the compound of the present invention may be used concomitantly with other agents such as phosphodiesterase 4 (PDE 4) inhibitors, bisphosphonate preparations, vitamin D preparations, calcium preparations, estrogen preparations, calcitonin preparations, ipriflavone preparations, protein assimilation steroids, vitamin K preparations, cathepsin K inhibitors, HMG-CoA reductase inhibitors, parathyroid hormones, growth factors, caspase-1 inhibitors, PTHrP derivatives, metalloproteinase inhibitors, farnesoid X receptor agonists, anti-androgen agents, selective estrogen receptor modulators (SERMs), progesterone agonists, calcium receptor antagonists (calcilitics), strontium preparations, α-calcitonin gene-related peptide preparations, bone morphogenetic protein preparations, anti-RANKL antibodies, anti-TNF-α antibodies, anti-IL-6 antibodies and the like. The agents which are used in combination may be contained in the percutaneous absorption preparations of the present invention. Additionally, administration method of the agents which are used in combination is not particularly limited and is either oral administration or parenteral administration.

As the phosphodiesterase 4 inhibitors, for example, cilomilast, roflumilast, alophyrin, OPC-6535, ONO-6126, IC-485, AWD-12-281, CC-10004, CC-1088, KW-4490, ZK-117137, YM-976, BY-61-9987, CC-7085, CDC-998, MEM-1414, ND-1251, Bay 19-8004, D-4396, PD-168787, atizoram, cipamfilin, rolipram, NIK-616, SCH-351591, V-11294A, OS-0217, GRC 3886, NIK-639 and the like can be cited.

Examples of the bisphosphonate preparations include sodium alendronate hydrate, ibandronic acid, disodium icandronate, disodium etidronate, olpadronate, sodium clodronate hydrate, zoledronic acta, disodium tiludronate, neridronate, disodium pamidronate, piridronate, minodronic acid hydrate, sodium risedronate hydrate, YM 175 and the like.

Examples of the vitamin D preparations include alfacalcidol, falecalcitriol, calcitriol, 1α,25-dihydroxycholecalciferol, dihydrotachysterol, ST-630, KDR, ED-71, rocaltrol (Ro 44-7190), tacalciol, maxacalcitol and the like.

Examples of the calcium preparations include calcium chloride, calcium gluconate, calcium glycerophosphate, calcium lactate, calcium L-aspartate, calcium phosphate dibasic and the like.

Examples of the estrogen preparations include estradiol, estradiol benzoate, estradiol cypionate, estradiol dipropionate, estradiol oenanthate, estradiol hexahydrobenzoate, estradiol phenylpropionate, estradiol undecanoate, estradiol valerate, estrone, ethinylestradiol, mestranol and the like.

Examples of the calcitonin preparations include calcitonin, sermon calcitonin (STH-32, SMC 20-51), chicken calcitonin (MGI-536), secalciferol, elcatonin, TJN-135 and the like.

Examples of the ipriflavone preparations include ipriflavone and the like.

Examples of the protein assimilation steroids include oxymetholone, stanozolol, nandrolone decanoate, nandrolone phenylpropionate, nandrolone cyclohexylpropionate, metenolone acetate, mestanolone, ethylestrenol, calusterone and the like.

Examples of the vitamin K preparations include menatetrenone, phytonadione and the like.

Examples of the cathepsin K inhibitors include ONO-5334, AAE 581, SB 462795 and the like.

Examples of the HMG-CoA reductase inhibitors include pravastatin sodium, simvastatin, fluvastatin sodium, cerivastatin sodium, itavastatin, atorvastatin calcium hydrate, lovastatin, pitavastatin calcium, DZ-4522, rosuvastatin and the like.

Examples of the parathyroid hormone (PTH) include dried thyroid, levothyroxine sodium, liothyronine sodium, propylthiouracil, thiamazole, teriparatide acetate and the like.

Examples of the growth factors include fibroblast growth factor (FGF), vascular endothelial growth factor (VEGF), hepatocyte growth factor (HGF), insulin-like growth factor (IGF) and the like.

Examples of the caspase-1 inhibitors include nitroflubiprofen, pralnacasan and the like.

Examples of the PTHrP derivatives include hPTHrP, RS-66271 and the like.

Examples of the metalloproteinase inhibitors include ONO-4817 and the like.

Examples of the farnesoid X receptor agonists include SR-45023A and the like.

Examples of the anti-androgen agents include osateron acetate and the like.

Examples of the selective estrogen receptor modulators include TSE-424, WJ-713/MPA, lasofoxifene tartrate, raloxifene hydrochloride, tamoxifen citrate and the like.

Examples of the progesterone agonists include trimegestone and the like.

Examples of the calcium receptor antagonists include NPS-423557 and the like.

Examples of the strontium preparations include strontium ranelate and the like.

Examples of the anti-RANKL antibodies include AMG 162 and the like.

Examples of the bone morphogenetic protein preparations include YM 484 and the like.

Examples of the anti-TNF-α antibodies include infliximab, etanercept, adalimumab and the like.

The other agents which is used in combination and described above are examples and not limited thereto.

The other agents may be administered in combination of two or more of optional species. Additionally, not only the substances so far found but also those which will be found in the future based on the aforementioned mechanism are included in the other agents which is used in combination.

### Examples

The following describes the present invention in detail with reference to examples, but the present invention is not limited thereto. Additionally, they may be changed within the scope which does not depart from the scope of the present invention.

### Preparation Examples

### Example 1

An adhesive liquid was prepared by dissolving a styrene-isoprene-styrene block copolymer (to be referred to as SIS hereinafter: SIS-5229, JSR) (300 mg), an ultra-hypochromic rosin ester (KE-311, manufactured by Arakawa Chemical Industries) (300 mg) and a light liquid paraffin (No. 70-S, manufactured by Sanko Kagaku Kogyo) (400 mg) in ethyl acetate (Kishida Chemical Co., Ltd.) (1000 mg). A coating liquid was prepared by dissolving methyl 4-{[2-((1R,2R,3R)-3-hydroxy-2-{(1E,3S)-3-hydroxy-4-[3-(methoxymethyl)phenyl]but-1-enyl}-5-oxocyclopentyl)ethyl]sulfanyl}butanoate (to be referred to compound A hereinafter) (40 mg) or PGE₁ (40 mg) in the adhesive liquid. The coating liquid was spread on a backing layer (a polyethylene film CoTrans 9720, 3M Health Care) to a thickness of about 60 µm using a Baker type applicator (Tester Sangyo Co., Ltd.). The adhesive face was dried under a reduced pressure at room temperature for 18 hours. The dried adhesive face was covered with a release liner and cut into a circle of 11 mm in diameter (0.95 cm²) (pharmaceutical preparation 1A) or 25 mm in diameter (4.9 cm²) (pharmaceutical preparation 1B) or a square of 10 cm² (3.2x3.2 cm) (pharmaceutical preparation 1C), to obtain pharmaceutical preparations (the main component content: 0.2 mg/cm²).

### Example 2

Pharmaceutical preparations (the main component content: 0.2 mg/cm⁻) were obtained by carrying out the same operation of the method shown in Example 1 using compound A and the adhesives, percutaneous permeation accelerators and other bases for external preparations shown in the following Table 1 (however, when the S1S and ultra-hypochromic rosin ester were used, ethyl acetate was added as the organic solvent (1000 mg when compound A was 40 mg, 2000 mg when compound A was 80 mg was added)) and cutting each product into a circle of 25 mm in diameter (about 4.9 cm²) (pharmaceutical preparation 2A) or a square of 3.2×3.2 cm (about 10 cm²) (pharmaceutical preparation 2B).

**Table 1**

| Examples | Compound A | Bases for external preparations | | |
|---|---|---|---|---|
| | | Adhesives | Percutaneous permeation accelerators | Others |
| 2(1) | 40 mg | SIS (300 mg) | OA (208 mg) | Light liquid |
| | | Rosin ester | CT (104 mg) | paraffin |
| | | (300 mg) | | (88 mg) |
| 2(2) | 40 mg | SIS (300 mg) | IPM (208 mg) | Light liquid |
| | | Rosin ester | CT (104 mg) | paraffin |
| | | (300 mg) | | (88 mg) |
| 2(3) | 80 mg | SIS (600 mg) | IPM (208 mg) | Light liquid |
| | | Rosin ester | CT (20.8 mg) | paraffin |
| | | (600 mg) | | (88 mg) |
| 2(4) | 40 mg | PE-300 | IPM (208 mg) | - |
| | | (688 mg) | CT (104 mg) | |
| 2(5) | 40 mg | PE-300 | IPM (104 mg) | - |
| | | (885.6 mg) | CT (10.4 mg) | |
| 2(6) | 40 mg | TS-620 | IPM (208 mg) | - |
| | | (688 mg) | CT (104 mg) | |
| 2(7) | 80 mg | SIS (600 mg) | IPM (416 mg) | Light liquid |
| | | Rosin ester | CT (204 mg) | paraffin |
| | | (600 mg) | | (176 mg) |
| 2(8) | 80 mg | SIS (600 mg) | IPM (208 mg) | Light liquid |
| | | Rosin ester | CT (20.8 mg) | paraffin |
| | | (600 mg) | | (571 mg) |
| 2(9) | 40 mg | PE-300 | - | - |
| | | (1000 mg) | | |

The bases for external preparations in the table represent the following meanings.
SIS: styrene-isoprene-styrene block copolymer (SIS-5229, JSR)
Rosin ester: ultra-hypochromic rosin ester (KE-311, Arakawa Chemical
Industries)
OA: oleic acid (Wako Pure Chemical Industries)
IPM: isopropyl myristate (Wako Pure Chemical Industries)
CT: crotamiton (Kongo Kagaku)
Light liquid paraffin (No. 70-S, Sanko Chemical Industries Co., Ltd.) PE-300: Nissetsu PE-300 (acrylic acid ester resin, Nippon Carbide Industries)
TS-620: Nikasol TS-620 (acrylic system copolymer resin, Nippon Carbide Industries)

### Example 3

Adhesive liquid was prepared by dissolving SIS (577 mg), an ultra-hypochromic rosin ester (KE-311, Arakawa Chemical Industries) (577 mg) and a light liquid paraffin (No. 70-S, Sanko Chemical Industries Co., Ltd.) (626 mg) in ethyl acetate (Kishida Chemical Cp., Ltd.) (2000 mg). A coating liquid was prepared by dissolving oleyl alcohol (AO-855 (trade name), NOF Corporation) (200 mg) and compound A (40 mg) in the adhesive liquid. The coating liquid was spread on a backing layer (a polyethylene terephthalate film) to be a thickness of about 60 µm using a Baker type applicator (Tester Sangyo Co., Ltd.). The adhesive face was dried under a reduced pressure at room temperature for 18 hours. The dried adhesive face was covered with a release liner and cut into a circle of 1.78 cm² to obtain a pharmaceutical preparation for patches (the main component content: 70 µg/cm²).

### Example 4

An adhesive liquid was prepared by dissolving SIS (1731 mg), an ultra-hypochromic rosin ester (KE-311, Arakawa Chemical Industries) (1731 mg) and a light liquid paraffin (No. 70-S, Sanko Chemistry Industry Co., Ltd.) (2478 mg) in ethyl acetate (Kishida Chemicals Co., Ltd.) (6000 mg). A coating liquid was prepared by dissolving compound A (60 mg) in the adhesive liquid. The coating liquid was spread on a backing layer (a polyethylene terephthalate film) to be a thickness of about 60 µm using a Baker type applicator (Tester Sangyo Co., Ltd.). The adhesive face was dried at room temperature for 1 minute using a dryer. The dried adhesive face was covered with a release liner and cut into a square or about 4 cm to obtain a pharmaceutical preparation for patches (the main component content: 70 µg/cm²).

### Example 5

An adhesive liquid was prepared by dissolving SIS (1731 mg), an ultra-hypochromic rosin ester (KE-311 (trade name), Arakawa Chemical Industries) (1731 mg) and a light liquid paraffin (No. 70-S (trade name), Sanko Chemistry Industry Co., Ltd.) (1878 mg) in ethyl acetate (Kishida Chemicals Co., Ltd.) (6000 mg). Additionally, a coating liquid was prepared by dissolving oleyl alcohol (AO-85S (trade name), NOF Corporation) (600 mg) and compound A (60 mg) in the adhesive liquid. The coating liquid was spread on a backing layer (a polyethylene terephtalate film) to be a thickness of about 60 µm using a Baker type applicator (Tester Sangyo Co., Ltd.). The adhesive face was dried at room temperature for 1 minute using a dryer. The dried adhesive face was covered with a release liner and cut into a square of about 4 cm² to obtain a pharmaceutical preparation for patches (the main component content: 70 µg/cm²).

### Example 6

An adhesive liquid was prepared by dissolving SIS (4 g), a high molecular weight polyisobutylene (Vistanex MML-10 (trade name), Exxon Mobile Chemical Company) (0.6 g), a low molecular weight polyisobutylene (Oppanol B12SPN (trade name), BASF Japan) (1.4 g), an ultra-hypochromic rosin ester (KE-311 (trade name), Arakawa Chemical Industries) (0.8 g), an alicyclic saturated hydrocarbon resin (Arcon P-100 (trade name), Arakawa Chemical Industries) (5.2 g), a liquid paraffin (Hicall M 352 (trade name), Kaneda) (7.56 g) and di-t-butylhydroxytoluene (Yoshinox (trade name), API Corporation) (0.04 g) in n-hexane (20 g). A coating liquid was prepared by dissolving compound A (0.4 g) in the adhesive liquid. The coating liquid was spread on a backing layer (a polyethylene terephthalate film) to be a thickness of about 60 µm using an applicator. The adhesive face was dried at room temperature. The dried adhesive face was covered with a release liner and cut into a square of about 4 cm² to obtain a pharmaceutical preparation for patches (the main component content: 140 µg/cm²).

### Example 7

An adhesive liquid was prepared by dissolving SIS (4 g), a high molecular weight polyisobutylene (Vistanex MML-10 (trade name), Exxon Mobile Chemical Company) (0.6 g), a low molecular weight polyisobutylene (Oppanol B12SPN (trade name), BASF Japan) (1.4 g), an ultra-hypochromic rosin ester (KE-311 (trade name), Arakawa Chemical Industries) (0.8 g), an alicyclic saturated hydrocarbon resin (Arcon P-100 (trade name), Arakawa Chemical Industries) (5.2 g), a liquid paraffin (Hicall M 352 (trade name), Kaneda) (6.56 g) and di-t-butylhydroxytoluene (Yoshinox (trade name), API Corporation) (0.04 g) in n-hexane (20 g). Also, a coating liquid was prepared by dissolving oleyl alcohol (AO-85S (trade name), NOF Corporation) (1 g) and compound A (0.4 g) in the adhesive liquid. The coating liquid was spread on a backing layer (a polyethylene terephthalate film) to oe a thickness or about 60 µm using an applicator. The adhesive face was dried at room temperature. The dried adhesive face was covered with a release liner and cut into a square of about 4 cm² to obtain a pharmaceutical preparation for patches (the main component content: 140 µg/cm²).

### Example 8

An adhesive liquid was prepared by dissolving SIS (4 g), a high molecular weight polyisobutylene (Vistanex MML-10 (trade name), Exxon Mobile Chemical Company) (0.6 g), a low molecular weight polyisobutylene (Oppanol B12SPN (trade name), BASF Japan) (1.4 g), an ultra-hypochromic rosin ester (KE-311 (trade name), Arakawa Chemical Industries) (0.8 g), an alicyclic saturated hydrocarbon resin (Arcon P-100 (trade name), Arakawa Chemical Industries) (5.2 g), a liquid paraffin (Hicall M 352 (trade name), Kaneda) (7.90 g) and di-t-butylhydroxytoluene (Yoshinox (trade name), API. Corporation) (0.04 g) in n-hexane (20 g). A coating liquid was prepared by dissolving compound A (0.06 g) in the adhesive liquid. The coating liquid was spread on a backing layer (a polyethylene terephthalate film) to be a thickness of about 60 µm using an applicator. The adhesive face was dried at room temperature. The dried adhesive face was covered with a release liner and cut into a square of about 4 cm² to obtain a pharmaceutical preparation for patches (the main component content: 30 µg/cm²).

### Example 9

An adhesive liquid was prepared by dissolving SIS (4 g), a high molecular weight polyisobutylene (Vistanex MML-10 (trade name), Exxon Mobile Chemical Company) (0.6 g), a low molecular weight polyisobutylene (Oppanol B12SPN (trade name), BASF Japan) (1.4 g), an ultra-hypochromic rosin ester (KE-311 (trade name), Arakawa Chemical Industries) (0.8 g), an alicyclic saturated hydrocarbon resin (Arcon P100 (trade name), Arakawa Chemical Industries) (5.2 g), a liquid paraffin (Hicall M 352 (trade name), Kaneda) (7.76 g) and di-t-butylhydroxytoluene (Yoshinox (trade name), API Corporation) (0.04 g) in n-hexane (20 g). A coating liquid was prepared by dissolving compound A (0.20 g) in the adhesive liquid. The coating liquid was spread on a backing layer (a polyethylene terephthalate film) to be a thickness of about 60 µm using an applicator. The adhesive face was dried at room temperature. The dried adhesive face was covered with a release liner and cut out into a square of about 4 cm² to obtain a pharmaceutical preparation for patches (the main component content: 100 µg/cm²).

### Biological Examples

### Test Example (1): In vitro skin permeability test (1)

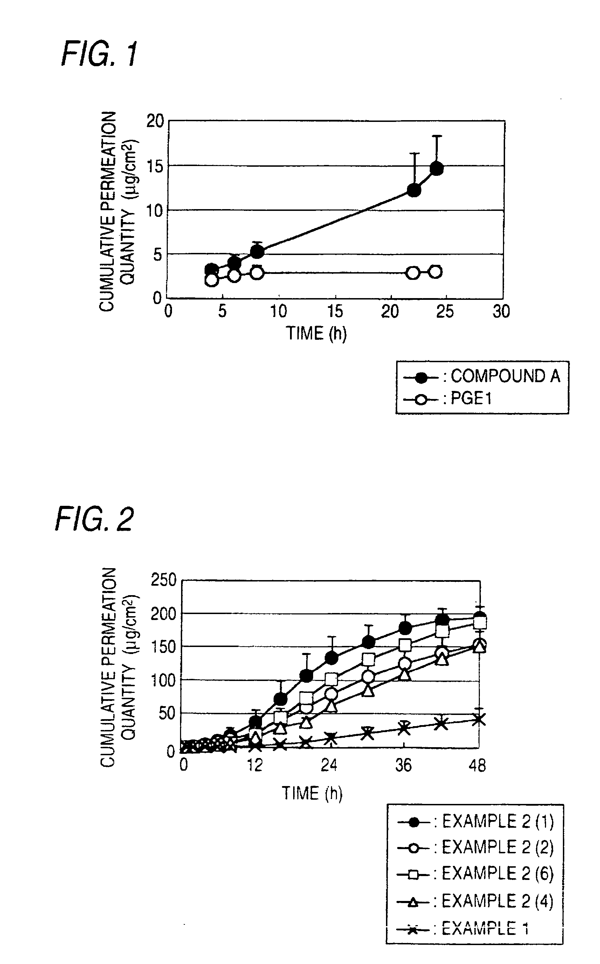

The hair-removed skin extracted from the abdominal region of each of male Wister rats (7 weeks of age, Wister/S1c, n = 3) was attached to a Franz type cell (1.1 cm in diameter). The aforementioned pharmaceutical preparation 1A prepared in Example 1 was applied to the stratum corneum of the hair-removed skin extracted from the abdominal region. Hanks' buffer (HBSS) (Sigma, pH 7.4) was applied to the dermal layer side cell. HBSS (200 µL) was periodically collected from the dermal layer side cell, and compound B as the active form of compound A or PGE₁ was determined by the HPLC method under the following conditions Cumulative permeation quantities until 24 hours were determined to calculate average values. The results are shown in Fig. 1.
HPLC conditions
Compound A (active form: compound B)
   Column: YMC-Pack ODS A-302 (4.6 mm 1.d., 150 mm),
   Column temperature: 50°C,
   Moving bed: 0.1% phosphoric acid aqueous solution: acetonitrile = 7:3,
   Detection wavelength 220 nm,
   Internal standard: ethyl p-hydroxybenzoate.
PGE₁
   Column: YMC-Pack ODS A-302 (4.6 mm 1.d., 150 mm),
   Column temperature: 30°C,
   Moving bed: 0.02 M potassium hydrogenphosphate aqueous solution:acetonitrile = 3:2,
   Detection wavelength: 205 nm,
   Internal standard: n-propyl p-hydroxybenzoate.

From this result, while high cumulative permeation quantity was not obtained by the patches of PGE₁, high cumulative permeation quantity was obtained by the patches of compound A. In this connection, since compound A was not detected in the dermal layer side cell, it was found that compound A was completely converted into the active form, compound B, during its permeation through the skin.

### Test Example (2): In vitro skin permeability test (2)

The skin extracted from the abdominal region of each of male hairless rats (7 weeks of age, HWY/SIc, n = 3) was attached to a Franz type cell (2.5 cm in diameter). The aforementioned pharmaceutical preparation 1B prepared in Example 1 or the pharmaceutical preparation 2A prepared in Example 2 (1), 2(2), 2(4) and 2(6) was applied to the stratum corneum of the skin extracted from the abdominal region. Physiological saline was applied to the dermal layer side cell. Physiological saline (200 µL) was periodically collected from the dermal layer side cell, and the compound B was determined by the HPLC method under the following conditions. Cumulative permeation quantities until 48 hours were determined to calculate average values. The results are shown in Fig. 2.
HPLC conditions
Column: YMC-Pack ODS A-302 (4.6 mm I.d., 150 mm),
Column temperature: a constant temperature at around 40°C,
Moving bed: 0.1% phosphoric acid aqueous solution: acetonitrile = 7:3,
Detection wavelength: 210 nm,
Internal standard: ethyl p-hydroxybenzoate.

From this result, cumulative permeation quantity of compound B was increased in comparison with the patches prepared in Example 1, by all combinations or the oases for external preparations

### Test Example (3): Measurement of blood concentration

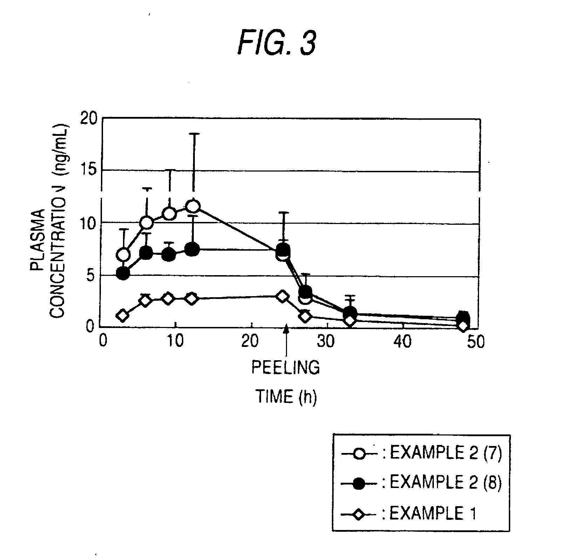

The abdominal skin of each of male hairless rats (10 weeks of age. HWY/SIc, n = 3) was shaved using an electric hair clipper. The aforementioned pharmaceutical preparation 1C prepared in Example 1 or the pharmaceutical preparation 2B prepared in Example 2 (7) and Example 2 (8) was applied to the abdominal region and blocked up for 24 hours by winding a non-woven pressure sensitive adhesive bandage (Mesh Pore, No. 50, manufactured by Nichiban Co., Ltd.). Each preparation was removed after 24 hours, and the administered region was lightly wiped up with absorbent cotton which was moistened with slightly warm water After 3, 6, 9, 12, 24, 27, 33 or 48 hours of the application, a blood sample (ca., 0.3 mL) was collected under non-anesthesia from the carotid artery using a heparin-treated polypropylene disposable syringe, and the blood plasma was transferred into a polypropylene container. The thus obtained plasma was immediately ice-cooled and subjected to cryopreservation. Thereafter, concentration of the compound B in plasma (ng/mL) at each period of time was measured, and average of 3 samples was calculated. The results are shown in Fig. 3.

From the results, the blood concentration of compound B in rats gradually reached 3 ng/ml in the case of the patches prepared in Inventive Example while the blood concentration of compound B reached from 5 ng/mL to 11 ng/mL during the 24 hours of application of the patches prepared in Example 2(7) and 2(8), and the blood concentration was further maintained continuously. Additionally, compound B was quickly disappeared from blood after peeling of the pharmaceutical preparations for patches.

### Test Example (4): Evaluation of primary skin irritation in hairless rats

In the Test Example (3), the patches produced in the aforementioned Inventive Examples were applied to the abdominal region of male hairless rats for 24 hours, and then skin reactions at the time of the removal of the patches (0 hour) and at the time of passage of 24 hours after their removal were observed and evaluated in accordance with the evaluation criteria of Draize "The Journal of Pharmacology and Experimental Therapeutics, vol. 83, pp 377 - 390, 1944" shown in Table 2.

**Table 2**

| A | Erythema and crust | Score |
|---|---|---|
| | No erythema | 0 |
| | Very slight erythema (barely observable degree) | 1 |
| | Evident erythema | 2 |
| | Medium to strong erythema | 3 |
| | Dark red and strong erythema with slight crust symptom (injury in deep region) | 4 |

| B | Edema | |
|---|---|---|
| | No edema | 0 |
| | Very slight edema (barely observable degree) | 1 |
| | Evident edema (clearly distinguishable from the periphery) | 2 |
| | Medium to strong edema (swelling of about 1 mm) | 3 |
| | Strong edema (swelling of 1 mm or more, widens toward the periphery too) | 4 |

Average score of each analyte was calculated. Additionally, average score of all animals was calculated from the average score of each analyte and used as the primary irritation index (PII).

Average score of each analyte = total of the score of each analyte/2 PII = total of the average score of each analyte/6

The safety plots employed are (i) no irritation when PII is 0, (ii) a weak irritant when PII exceeds 0 and is 2 or less, (iii) a medium irritant when it exceeds 2 and is 5 or less and (iv) a strong irritant when it exceeds 5.

As a result of this, the primary skin irritation index of each of the patches of the present invention was 1 or less. Therefore, it showed weak irritation and no problems. Especially, edema was not found at all. For example, in the case of the pharmaceutical preparation 2B prepared in Example 2(8), its PII was 0.67, and edema was not observed in the individuals.

### Stability Test

Each of the aforementioned patches prepared in Inventive Example 2(3), 2(9) and 2(10) was cut into a certain size (2x2 cm) and its mass was measured. Each sample was sealed by putting into an Aluminum Pillow together with silica gel (Dryern (registered trade mark), Yamani Yakuhin Co., Ltd.) and preserved for (1) 2 weeks, 1 month or 3 months under a condition of 5°C, (2) 2 weeks, 1 month or 3 months under a condition of 25°C or (3) 2 weeks, 1 month or 3 months under a condition of 40°C. Compound A in each sample after the preservation and each sample before the preservation was detected by the HPLC method under the conditions shown in Test Example (2).

As a result of this, they were stable under conditions of 25°C and 2 weeks in all combinations of the bases for external preparations. For example, survival rate of compound A after 25°C and 2 weeks was 97.5% in the case of the patches of Example 2(3).

### Test Example (5): Drug permeability test using human skin

Human skin purchased from Asterand (abdominal skin of a woman of 66 years old, epidermal-dermal skin (no heat treatment), n = 3) was attached to a Franz type cell (1.78 cm²) , and the pharmaceutical preparation produced in Example 3 was applied to the stratum corneum side. Physiological saline (8 ml) was applied to the dermal layer side cell. From the dermal layer side cell, 1 ml portion thereof was periodically collected, and the compound A and compound B were determined by LC/MS/MS method under the following conditions. Cumulative permeation quantities until 48 hours were determined to calculate average values.
LC/MS/MS measuring conditions (HPLC)
Measuring instrument: Perkin-Elmer Series 200 Micro-pumps and CTC Analytics AS 859 Autosampler
column: Keystone-Hypersil BDSC 1830×2.1 mm, 3 µm Eluents: Solvent ratio was periodically adjusted in accordance with the following Table 3.

**Table 3**

| Time | Liquid A | Liquid B |
|---|---|---|
| (minute) | (water:buffer = 90: 10) (%) | (acetonitrile: buffer = 90:10) (%) |
| 0.0 | 100 | 0 |
| 3.0 | 10 | 90 |
| 3.1 | 100 | 0 |
| 4.5 | 100 | 0 |

In this connection, the buffer in the Table represents 25 mM ammonium hydroxide aqueous solution (pH 3.5):formic acid = 15:85 (volume ratio).
Flow rate. 300 µl/minute
Injection quantity: 5 µl
Running time: 4.5 minutes
Retention time: compound A; until 3.45 minutes/compound B; until 3.25 minutes
Mass spectrometry
Apparatus: PE SCIEX API 3000
Interface: electron spray (turbo ion spray)
Mode: multiple reaction monitoring (MRM)

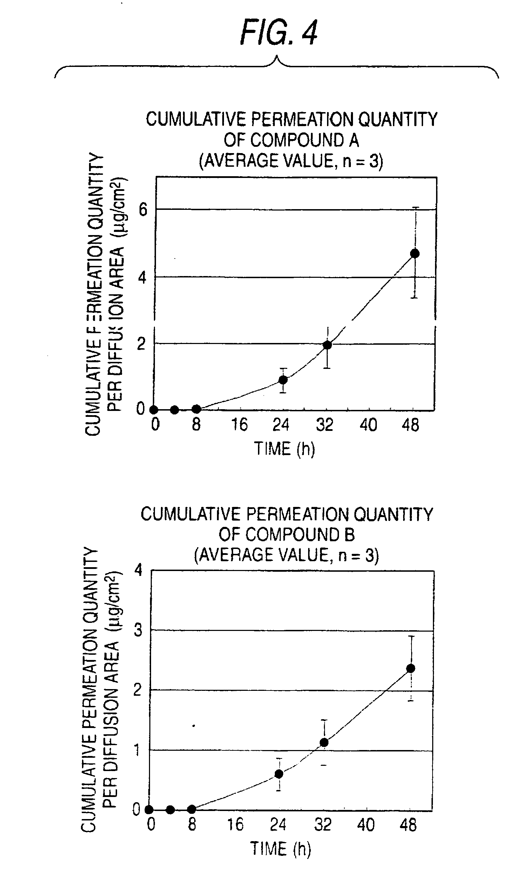

Human skin permeability of compound A and compound B which is its active form was measured to calculate the cumulative permeation quantities, with the results shown in Fig. 4. Their skin permeation rates were calculated from the slope of cumulative permeation quantities in Fig. 4. Compound A was 0.16 ± 0.04 (µg/cm²/hr), and compound B was 0.07 ± 0.01 (µg/cm²/hr).

Additionally, predicted human blood concentration was calculated by dividing "skin permeation rate" by "total body clearance". The predicted human blood concentration was 0.7 pg/mL by 1 cm² plastering, 3.7 pg/mL by 5 cm² plastering and 7.4 pg/mL by 10 cm² plastering.

In this connection, the total body clearance was calculated based on 5.180 L/hr-kg, body weight 60 kg.

### Industrial Applicability

The percutaneous absorption preparations of the present invention can stably maintain blood concentration of the active form of the present invention and are safe since there are no side effects. Therefore, they can be used as medicines.

### Brief Description of the Drawings

Fig. 1 is a result of the *in vitro* skin permeability test (1) in Test Example (1) of Biological Examples. The cumulative permeation quantity of compound A represents a value calculated by converting the detected value of compound B into compound A.
Fig. 2 is a result of the *in vitro* skin permeability test (2) in Test Example (2) of Biological Examples.
Fig. 3 is a result of the measurement of blood concentration in Test Example (3) of Biological Examples.
Fig. 4 is a result of a human skin permeability test which used the pharmaceutical preparations produced in Example 3. It represents cumulatively detected amounts of compound A and compound B.

## Claims

1. A blood concentration regulation type patch, which comprises methyl 4-{[2-((1R,2R,3R)-3-hydroxy-2-{(1E,3S)-3-hydroxy-4-[3-(methoxymethyl)phenyl]but-1-enyl}-5-oxocyclopentyl)ethyl]sulfanyl}butanoate, a percutaneous permeation accelerator which is one or more substances selected from the group consisting of isopropyl myristate, crotamiton, oleic acid and oleyl alcohol and an adhesive which may contain an adhesion providing agent, a softening agent, and/or an antioxidant.

2. The patch according to claim 1,
wherein the adhesive is one or more substances selected from the group consisting of a styrene-isoprene-styrene block copolymer, an acrylic acid ester resin and an acrylic system copolymer resin;
the adhesion providing agent is one or more substances selected from a group consisting of a rosin resin, an alicyclic saturated hydrocarbon resin and a polyisobutylene;
the softening agent is liquid paraffins; and
the antioxidant is di-t-butylhydroxytoluene.

3. The patch according to claim 2,
wherein the adhesive is a styrene-isoprene-styrene block copolymer;
the adhesion providing agent is one or more substances selected from the group consisting of a rosin resin, an alicyclic saturated hydrocarbon resin and a polyisobutylene; and
the percutaneous permeation accelerator is oleyl alcohol.

4. The patch according to claim 1, which is an agent for preventing, treating and/or advance-suppressing a bone disease.

5. The patch according to claim 4, wherein the bone disease is bone fracture or vertebral body fracture.

6. The patch according to any one of claims 1 to 5, which comprises (i) from about 10 parts by mass to about 200 parts by mass of a styrene-isoprene-styrene block copolymer, (ii) from about 20 parts by mass to about 300 parts by mass of an alicyclic saturated hydrocarbon resin, (iii) from about 30 parts by mass to about 500 parts by mass of liquid paraffins, (iv) from about 1 part by mass to about 100 parts by mass of a high molecular weight polyisobutylene, (v) from about 5 parts by mass to about 200 parts by mass of a low molecular weight polyisobutylene, (vi) from about 1 part by mass to about 100 parts by mass of a rosin resin and (vii) from about 0.1 part by mass to about 3 parts by mass of di-t-butylhydroxytoluene, and further comprises from about 1 part by mass to about 50 parts by mass of oleyl alcohol, based on 1 part by mass of methyl 4-{[2-((1R,2R,3R)-3-hydroxy-2-{(1E,3S)-3-hydroxy-4-[3-(methoxymethyl)phenyl]but-1-enyl}-5-oxocyclopentyl)ethyl]sulfanyl}butanoate.

7. The patch according to claim 6, wherein the methyl 4-{[2-((1R,2R,3R)-3-hydroxy-2-{(1E,3S)-3-hydroxy-4-[3-(methoxymethyl)phenyl]but-1-enyl}-5-oxocyclopentyl)ethyl]sulfanyl}butanoate content per one dose is from about 0.01 mg to about 3 mg.

8. The patch according to claims 6 or 7, which is adhered once a day or re-adhered once during 2 to 4 days.

9. A blood concentration regulation type patch which comprises methyl 4-{[2-((1R,2R,3R)-3-hydroxy-2-{(1E,3S)-3-hydroxy-4-[3-(methoxymethyl)phenyl]but-1-enyl}-5-oxocyclopentyl)ethyl]sulfanyl}butanoate, a percutaneous permeation accelerator which is one or more substances selected from the group consisting of isopropyl myristate, crotamiton, oleic acid and oleyl alcohol and an adhesive which may contain an adhesion providing agent, a softening agent and/or an antioxidant, for use in preventing, treating and/or advance-suppressing a bone disease.

## Patentansprüche

1. Pflaster vom die Konzentration im Blut regulierenden Typ, welches Methyl-4-{[2-((1R,2R,3R)-3-hydroxy-2-{(1E,3S)-3-hydroxy-4-[3-(methoxymethyl)phenyl]but-1-enyl}-5-oxocyclopentyl)ethyl]sulfanyl}butanoat, einen Beschleuniger der perkutanen Permeation, bei dem es sich um eine oder mehrere Substanzen handelt, die ausgewählt sind aus der Gruppe bestehend aus Isopropylmyristat, Crotamiton, Ölsäure und Oleylalkohol, und einen Klebstoff, welcher ein Haftmittel, ein Erweichungsmittel und/oder ein Antioxidationsmittel enthalten kann, umfasst.

2. Pflaster nach Anspruch 1,
wobei es sich bei dem Klebstoff um eine oder mehrere Substanzen handelt, die ausgewählt sind aus der Gruppe bestehend aus einem Styrol-Isopren-Styrol-Blockcopolymer, einem Acrylsäureesterharz und einem Acrylcopolymerharz;
es sich bei dem Haftmittel um eine oder mehrere Substanzen handelt, die ausgewählt sind aus einer Gruppe bestehend aus einem Kolophoniumharz, einem alicyclischen gesättigten Kohlenwasserstoffharz und einem Polyisobutylen;
es sich bei dem Erweichungsmittel um flüssige Paraffine handelt; und
es sich bei dem Antioxidationsmittel um Di-t-butylhydroxytoluol handelt.

3. Pflaster nach Anspruch 2,
wobei der Klebstoff ein Styrol-Isopren-Styrol-Blockcopolymer ist;
es sich bei dem Haftmittel um eine oder mehrere Substanzen handelt, die ausgewählt sind aus der Gruppe bestehend aus einem Kolophoniumharz, einem alicyclischen gesättigten Kohlenwasserstoffharz und einem Polyisobutylen; und
der Beschleuniger der perkutanen Permeation Oleylalkohol ist.

4. Pflaster nach Anspruch 1, welches ein Mittel zum Verhüten, Behandeln und/oder Unterdrücken des Fortschreitens einer Knochenerkrankung ist.

5. Pflaster nach Anspruch 4, wobei es sich bei der Knochenerkrankung um einen Knochenbruch oder Wirbelkörperbruch handelt.

6. Pflaster nach einem der Ansprüche 1 bis 5, welches (i) ungefähr 10 Massenteile bis ungefähr 200 Massenteile eines Styrol-Isopren-Styrol-Blockcopolymers, (ii) ungefähr 20 Massenteile bis ungefähr 300 Massenteile eines alicyclischen gesättigten Kohlenwasserstoffharzes, (iii) ungefähr 30 Massenteile bis ungefähr 500 Massenteile flüssige Paraffine, (iv) ungefähr 1 Massenteil bis ungefähr 100 Massenteile eines Polyisobutylens mit hohem Molekulargewicht, (v) ungefähr 5 Massenteile bis ungefähr 200 Massenteile eines Polyisobutylens mit niedrigem Molekulargewicht, (vi) ungefähr 1 Massenteil bis ungefähr 100 Massenteile eines Kolophoniumharzes und (vii) ungefähr 0,1 Massenteil bis ungefähr 3 Massenteile Di-t-butylhydroxytoluol umfasst und außerdem ungefähr 1 Massenteil bis ungefähr 50 Massenteile Oleylalkohol umfasst, bezogen auf 1 Massenteil Methyl-4-{[2-((1R,2R,3R)-3-hydroxy-2-{(1E,3S)-3-hydroxy-4-[3-(methoxymethyl)phenyl]but-1-enyl}-5-oxocyclopentyl)ethyl]sulfanyl}butanoat.

7. Pflaster nach Anspruch 6, wobei der Methyl-4-{[2-((1R,2R,3R)-3-hydroxy-2-{(1E,3S)-3-hydroxy-4-[3-(methoxymethyl)phenyl]but-1-enyl}-5-oxocyclopentyl)ethyl]sulfanyl}butanoat-Gehalt pro eine Dosis ungefähr 0,01 mg bis ungefähr 3 mg beträgt.

8. Pflaster nach den Ansprüchen 6 oder 7, welches einmal täglich aufgeklebt oder einmal während 2 bis 4 Tagen wieder aufgeklebt wird.

9. Pflaster vom die Konzentration im Blut regulierenden Typ, welches Methyl-4-{[2-((1R,2R,3R)-3-hydroxy-2-{(1E,3S)-3-hydroxy-4-[3-(methoxymethyl)phenyl]but-1-enyl}-5-oxocyclopentyl)ethyl]sulfanyl}butanoat, einen Beschleuniger der perkutanen Permeation, bei dem es sich um eine oder mehrere Substanzen handelt, die ausgewählt sind aus der Gruppe bestehend aus Isopropylmyristat, Crotamiton, Ölsäure und Oleylalkohol, und einen Klebstoff, welcher ein Haftmittel, ein Erweichungsmittel und/oder ein Antioxidationsmittel enthalten kann, umfasst, zur Verwendung beim Verhüten, Behandeln und/oder Unterdrücken des Fortschreitens einer Knochenerkrankung.

## Revendications

1. Timbre transdermique de type à régulation de concentration sanguine, lequel comprend du 4-{[2-((1R,2R,3R)-3-hydroxy-2-{(1E,3S)-3-hydroxy-4-[3-(méthoxyméthyl)phényl]but-1-ényl}-5-oxocyclopentyl)éthyl]-sulfanyl}butanoate de méthyle, un accélérateur de perméation percutanée qui est une ou plusieurs substances choisies dans le groupe constitué du myristate d'isopropyle, du crotamiton, de l'acide oléique et de l'alcool oléylique et un adhésif qui peut contenir un agent fournissant une adhérence, un agent ramollissant et/ou un antixodyant.

2. Timbre transdermique selon la revendication 1,
dans lequel l'adhésif est une ou plusieurs substances choisies dans le groupe constitué d'un copolymère séquencé de styrène-isoprène-styrène, d'une résine d'ester d'acide acrylique et d'une résine copolymère de système acrylique ;
l'agent fournissant une adhérence est une ou plusieurs substances choisies dans un groupe constitué d'une résine de colophane; d'une résine hydrocarbonée saturée alicyclique et d'un polyisobutylène ;
l'agent ramollissant est des paraffines liquides ; et l'antioxydant est le di-t-butylhydroxytoluène.

3. Timbre transdermique selon la revendication 2,
dans lequel l'adhésif est un copolymère séquencé de styrène-isoprène-styrène ;
l'agent fournissant une adhérence est une ou plusieurs substances choisies dans le groupe constitué d'une résine de colophane, d'une résine hydrocarbonée saturée alicyclique et d'un polyisobutylène ; et
l'accélérateur de perméation percutanée est l'alcool oléylique.

4. Timbre transdermique selon la revendication 1, lequel est un agent pour éviter, traiter et/ou supprimer le développement d'une maladie osseuse.

5. Timbre transdermique selon la revendication 4, dans lequel la maladie osseuse est une fracture osseuse ou une fracture de corps vertébral.

6. Timbre transdermique selon l'une quelconque des revendications 1 à 5, lequel comprend (i) d'environ 10 parties en masse à environ 200 parties en masse d'un copolymère séquencé de styrène-isoprène-styrène, (ii) d'environ 20 parties en masse à environ 300 parties en masse d'une résine hydrocarbonée saturée alicyclique, (iii) d'environ 30 parties en masse à environ 500 parties en masse de paraffines liquides, (iv) d'environ 1 partie en masse à environ 100 parties en masse d'un polyisobutylène de masse moléculaire élevée, (v) d'environ 5 parties en masse à environ 200 parties en masse d'un polyisobutylène de faible masse moléculaire, (vi) d'environ 1 partie en masse à environ 100 parties en masse d'une résine de colophane et (vii) d'environ 0,1 partie en masse à environ 3 parties en masse de di-t-butylhydroxytoluène, et comprend de plus d'environ 1 partie en masse à environ 50 parties en masse d'alcool oléylique, rapporté à 1 partie en masse de 4-{[2-((1R,2R,3R)-3-hydroxy-2-{(1E,3S)-3-hydroxy-4-[3-(méthoxyméthyl)-phényl]but-1-ényl}-5-oxocyclopentyl)éthyl]sulfanyl}butanoate de méthyle.

7. Timbre transdermique selon la revendication 6, dans lequel la teneur en 4-{[2-((1R,2R,3R)-3-hydroxy-2-{(1E,3S)-3-hydroxy-4-[3-(méthoxyméthyl)phényl]but-1-ényl}-5-oxocyclopentyl)éthyl]sulfanyl}butanoate de méthyle pour une dose est d'environ 0,01 mg à environ 3 mg.

8. Timbre transdermique selon la revendication 6 ou 7, lequel est collé une fois par jour ou est recollé une fois pendant de 2 à 4 jours.

9. Timbre transdermique de type à régulation de concentration sanguine qui comprend du 4-{[2-((1R,2R,3R)-3-hydroxy-2-{(1E,3S)-3-hydroxy-4-[3-(méthoxyméthyl)phényl]but-1-ényl}-5-oxocyclopentyl)éthyl]-sulfanyl}butanoate de méthyle, un accélérateur de perméation percutanée qui est une ou plusieurs substances choisies dans le groupe constitué du myristate d'isopropyle, du crotamiton, de l'acide oléique et de l'alcool oléylique, un adhésif qui contient un agent fournissant une adhérence, un agent ramollissant et/ou un antioxydant, pour une utilisation destinée à éviter, à traiter et/ou à supprimer le développement d'une maladie osseuse.
